(19) Europäisches Patentamt
European Patent Office
Office européen des brevets

(11) **EP 4 670 641 A1**

(12) **EUROPEAN PATENT APPLICATION**

(43) Date of publication:
**31.12.2025 Bulletin 2026/01**

(21) Application number: **25185160.6**

(22) Date of filing: **25.06.2025**

(51) International Patent Classification (IPC):
***A61B 6/50*** *(2024.01)* ***A61B 6/03*** *(2006.01)*

(52) Cooperative Patent Classification (CPC):
**A61B 6/507; A61B 6/032; A61B 6/481;**
**A61B 6/482; A61B 6/486; A61B 6/5217;**
**A61B 6/542;** A61B 6/4014; A61B 6/503

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **25.06.2024 CN 202410834025**

(71) Applicant: **Shanghai United Imaging Healthcare Co., Ltd.**
**Shanghai 201807 (CN)**

(72) Inventor: **ZHAO, Xiaofen**
**Shanghai 201807 (CN)**

(74) Representative: **Wang, Bo**
**Panovision IP**
**Ebersberger Straße 3**
**85570 Markt Schwaben (DE)**

(54) **METHODS, DEVICES, AND SYSTEMS FOR CT SCANNING**

(57) Methods, devices, and systems for CT scanning are provided. The methods include obtaining a scanning instruction for a target object. The scanning instruction is configured to instruct to perform a combination of spectrum CT and CT perfusion scanning on the target object. The scanning instruction includes scanning time information of the combination of spectrum CT and CT perfusion scanning. The scanning time information includes a spectrum scanning phase and a perfusion scanning phase, and the spectrum scanning phase is one or more scanning phases of the perfusion scanning phase. The methods further include in response to the scanning instruction, performing a CT perfusion scanning on the target object in the perfusion scanning phase other than the spectrum scanning phase, and performing a spectrum CT scanning on the target object in the spectrum scanning phase.

<u>**400**</u>

FIG. 4

**Description**

**CROSS REFERENCE TO RELATED APPLICATIONS**

This application claims priority to Chinese Patent Application No. 202410834025.5, filed on June 25, 2024, and the entire content of which is incorporated herein by reference.

**TECHNICAL FIELD**

The present disclosure relates to the field of computed tomography (CT) technology, and in particular, to a method, device, and system for CT scanning.

**BACKGROUND**

Computed tomography (CT) is a widely utilized medical imaging modality. Within CT imaging, material differentiation is achieved via spectral imaging techniques, whereas CT perfusion imaging provides information regarding tissue and organ blood supply. Blood supply, comprising vascular structures such as blood vessels and lymphatic vessels, constitutes a fundamental component of human tissues. A combination of spectrum CT and CT perfusion scanning (also termed "one-stop spectrum CT and CT perfusion scanning" or "one-stop perfusion and angiographic imaging") refers to a method for concurrently acquiring both perfusion data and spectral data during a single scan session. Conventional approaches for combining spectrum CT and CT perfusion scanning typically employ multi-phase or segmented scanning protocols. For instance, the overall scan protocol may be segmented into three distinct phases: a first phase and a second phase utilize perfusion mode scanning to generate perfusion images, while a third phase employs spectral mode scanning to obtain spectral images. Thus, it is evident that existing multi-phase scanning fundamentally executes each imaging mode sequentially. Consequently, data acquisition for one imaging mode is precluded during the phases dedicated to the other mode. This limitation diminishes the diagnostic information content available from the acquired images and adversely impacts diagnostic efficacy.

Therefore, it is desired to provide a method, device, and system for CT scanning that enhances the completeness of information in both perfusion images and spectrum images, thereby improving clinical diagnostic effectiveness.

**SUMMARY**

Embodiments of the present disclosure provide a method for CT scanning. The method is executed by a computing device including at least one processor and at least one storage device. The method comprises obtaining a scanning instruction for a target object, the scanning instruction is configured to instruct to perform a combination of spectrum CT and CT perfusion scanning on the target object. The scanning instruction includes scanning time information of the combination of spectrum CT and CT perfusion scanning. The scanning time information includes a spectrum scanning phase and a perfusion scanning phase, and the spectrum scanning phase is one or more scanning phases of the perfusion scanning phase. The method comprises, in response to the scanning instruction, performing a CT perfusion scanning on the target object in the perfusion scanning phase other than the spectrum scanning phase, and performing a spectrum CT scanning on the target object in the spectrum scanning phase.

In some embodiments, the scanning instruction further includes energy information of the combination of spectrum CT and CT perfusion scanning, the energy information includes a single-energy voltage and a dual-energy voltage. The performing the CT perfusion scanning on the target object in the perfusion scanning phase and the performing the spectrum CT scanning on the target object in the spectrum scanning phase includes: obtaining, using a perfusion scanning mode, single-energy scanning data of a region of interest (ROI) of the target object by emitting single-energy rays based on the single-energy voltage on the target object in the perfusion scanning phase other than the spectrum scanning phase, and obtaining dual-energy scanning data of the ROI of the target object by emitting dual-energy rays based on the dual-energy voltage on the target object in the spectrum scanning phase.

In some embodiments, the method further includes generating a perfusion analysis result of the ROI based on the single-energy scanning data and the dual-energy scanning data.

In some embodiments, the spectrum scanning phase is a portion of the perfusion scanning phase, and the generating a perfusion analysis result of the ROI based on the single-energy scanning data and the dual-energy scanning data includes: generating a perfusion image of the ROI based on first single-energy scanning data in the perfusion scanning phase other than the spectrum scanning phase and second single-energy scanning data of the dual-energy scanning data; generating a spectrum image of the ROI based on the dual-energy scanning data; and obtaining the perfusion analysis result based on the spectrum image and the perfusion image. The obtaining the perfusion analysis result includes obtaining the perfusion analysis result by performing a perfusion analysis based on the spectrum image and the perfusion image in the perfusion scanning phase other than the spectrum scanning phase; or obtaining the perfusion analysis result by performing a conjoint perfusion analysis on the spectrum image and the perfusion image.

In some embodiments, the obtaining the perfusion analysis result by performing a perfusion analysis based on the spectrum image and the perfusion image in the perfusion scanning phase other than the spectrum scanning phase includes: obtaining third single-energy scanning data by performing a scanning data conversion on the spectrum image; and obtaining the perfusion analysis result by performing the perfusion analysis based on the first single-energy scanning data and the third single-energy scanning data.

In some embodiments, the spectrum scanning phase is identical to the perfusion scanning phase, and the generating a perfusion analysis result of the ROI based on the single-energy scanning data and the dual-energy scanning data includes: obtaining fourth single-energy scanning data of the spectrum scanning phase based on the dual-energy scanning data and generating a perfusion image of the ROI based on the fourth single-energy scanning data; and/or generating a spectrum image of the ROI based on the dual-energy scanning data.

In some embodiments, the method further comprises obtaining the perfusion analysis result by performing a conjoint perfusion analysis on the spectrum image and the perfusion image.

In some embodiments, the method further comprises obtaining the perfusion analysis result by performing a perfusion analysis on the spectrum image or the perfusion image.

In some embodiments, the obtaining the perfusion analysis result by performing a perfusion analysis on the spectrum image or the perfusion image includes: extracting a characteristic parameter of the spectrum image, the characteristic parameter characterizing absorption properties of each substance in the ROI for rays of different energies; establishing a time attenuation curve based on the characteristic parameter; and obtaining the perfusion analysis result by performing the perfusion analysis on the time attenuation curve.

In some embodiments, the spectrum scanning phase is identical to the perfusion scanning phase, and the generating a perfusion analysis result of the ROI based on the single-energy scanning data and the dual-energy scanning data includes: obtaining spectrum data and perfusion data by performing a spectrum analysis and processing on the dual-energy scanning data; obtaining a spectrum perfusion image by fusing the spectrum data and the perfusion data; and generating the perfusion analysis result by performing a perfusion analysis on the spectrum perfusion image.

In some embodiments, the generating the perfusion analysis result by performing the perfusion analysis on the spectrum perfusion image includes: extracting a characteristic parameter of the spectrum image, the characteristic parameter characterizing absorption properties of each substance in the ROI for rays of different energies; establishing a time attenuation curve based on the characteristic parameter; and obtaining the perfusion analysis result by performing the perfusion analysis on the time attenuation curve.

Embodiments of the present disclosure provide a device for CT scanning, comprising at least one processor, and the at least one processor is configured to execute the method described above.

Embodiments of the present disclosure provide a method for CT scanning, executed by a computing device including at least one processor and at least one storage device. The scanning method comprises: in response to an operation of a user on an interactive interface of a CT system, generating a scanning instruction for a target object. The scanning instruction indicates a scanning mode set by the user, and the scanning mode includes dual-energy scanning or a combination of single-energy scanning and dual-energy scanning. The scanning method comprise: in response to determining that the target object is injected with a contrast agent, controlling the CT system to execute the scanning instruction to obtain scanning data. An execution process of the scanning instruction is divided into a plurality of phases according to a dose of the contrast agent in a region of interest (ROI) of the target object, and during at least one phase of the plurality of phases, the dual-energy scanning is performed to acquire dual-energy scanning data. The scanning method comprises obtaining an angiographic image of the at least one phase of the target object based on the dual-energy scanning data.

Embodiments of the present disclosure provide a method for CT scanning, executed by a computing device including at least one processor and at least one storage device. The scanning method comprises: in response to determining that a target object is injected with a contrast agent, controlling a CT system to execute a scanning instruction to obtain scanning data. An execution process of the scanning instruction is divided into a plurality of phases according to a dose of the contrast agent in a region of interest (ROI) of the target object. During at least one first phase of the plurality of phases, single-energy scanning is performed to obtain single-energy scanning data, and during at least one second phase of the plurality of phases, dual-energy scanning is performed to obtain dual-energy scanning data. The scanning data includes the single-energy scanning data and the dual-energy scanning data. The scanning method comprises: in response to an operation of a user on an interactive interface of the CT system, selecting a reconstruction mode. The reconstruction mode includes at least one of an angiography reconstruction mode, a perfusion imaging reconstruction mode, or a spectral imaging reconstruction mode. The scanning method comprises obtaining a target image of the target object by reconstruction based on the reconstruction mode and the scanning data.

Embodiments of the present disclosure provide a system for CT scanning. The system comprises two radiation sources, the two radiation sources including a first radiation source and a second radiation source. The first radiation source is configured to generate low energy X-rays based on a first voltage, and the second radiation source is configured to generate high energy X-rays based on a second voltage. The system comprises two detector arrays, corresponding to the two radiation sources, respectively. The two detector arrays are configured to receive X-rays transmitted through a target object and generate scanning data representing the X-rays transmitted through the target object. The system comprises a terminal including an interactive interface configured to interact with a user and at least one processor and at least one storage device, the at least one storage device storing computer instructions. When the computer instructions are executed by the at least one processor, the at least one processor executes following operations: in response to an operation of the user on the interactive interface, generating a scanning instruction for the target object. The scanning instruction indicates a scanning mode set by the user, and the scanning mode includes dual-energy scanning or a combination of single-energy scanning and dual-energy scanning. During the single-energy scanning, the first radiation source works, and during the dual-energy scanning, the first radiation source and the second radiation source work simultaneously. The at least one processor executes: in response to determining the target object is injected with a contrast agent, controlling the two radiation sources to execute the scanning instruction, and controlling the two detector arrays to acquire scanning data of a heart of the target object. An execution process of the scanning instruction is divided into a plurality of phases according to a dose of the contrast agent in the heart, and during at least one phase of the plurality of phases, the dual-energy scanning is performed. The at least one processor executes: obtaining at least one of: a high-energy myocardial vascular image, a low-energy myocardial vascular image, a virtual single-energy image, a spectrum curve, or a perfusion image based on the scanning data, and displaying the obtained image on the interactive interface.

Embodiments of the present disclosure provide a system for CT scanning. The system comprises two radiation sources, the two radiation sources including a first radiation source and a second radiation source. The first radiation source is configured to generate low energy X-rays based on a first voltage, and the second radiation source is configured to generate high energy X-rays based on a second voltage. The system comprises two detector arrays, corresponding to the two radiation sources, respectively. The two detector arrays are configured to receive X-rays transmitted through a target object and generate scanning data representing the X-rays transmitted through the target object. The system comprises a terminal, including an interactive interface configured to interact with a user and at least one processor and at least one storage device, the at least one storage device storing computer instructions. When the computer instructions are executed by the at least one processor, the at least one processor executes following operations: in response to an operation of the user on the interactive interface, generating a scanning instruction for the target object. The scanning instruction indicates a scanning mode set by the user, and the scanning mode includes dual-energy scanning. During the dual-energy scanning, the first radiation source and the second radiation source work simultaneously. The at least one processor executes: in response to determining that a bone tissue of the target object is injected with a contrast agent, controlling the two radiation sources to execute the scanning instruction, and controlling the two detector arrays to acquire scanning data of the bone tissue. An execution process of the scanning instruction is divided into a plurality of phases according to a dose of the contrast agent in the bone tissue. The at least one processor executes: obtaining iodine concentration images of the plurality of phases based on the scanning data, and obtaining a perfusion analysis result of the bone tissue based on the iodine concentration images of the plurality of phases.

**BRIEF DESCRIPTION OF THE DRAWINGS**

The present disclosure is further describable in terms of exemplary embodiments. These exemplary embodiments are describable in detail with reference to the drawings. These embodiments are non-limiting exemplary embodiments, in which like reference numerals represent similar structures throughout the several views of the drawings:

FIG. 1 is a schematic diagram illustrating an exemplary computed tomography (CT) system according to some embodiments of the present disclosure;
FIG. 2 is a schematic diagram illustrating an exemplary imaging device in FIG. 1 according to some embodiments of the present disclosure;
FIG. 3 is a schematic diagram illustrating an exemplary CT system according to some embodiments of the present disclosure;
FIG. 4 is a flowchart illustrating an exemplary process of CT scanning according to some embodiments of the present disclosure;
FIG. 5 is a schematic diagram illustrating an exemplary interactive interface of a CT system according to some embodiments of the present disclosure;
FIG. 6 is a flowchart illustrating an exemplary process of CT scanning according to some embodiments of the present disclosure;
FIG. 7 is a flowchart illustrating an exemplary process of CT scanning according to some embodiments of the present disclosure;
FIG. 8 is a flowchart illustrating an exemplary process of CT scanning according to some embodiments of the present disclosure;

FIG. 9 is a flowchart illustrating an exemplary process of CT scanning according to some embodiments of the present disclosure;

FIG. 10 is a flowchart illustrating an exemplary process of CT scanning according to some embodiments of the present disclosure;

FIG. 11 is a flowchart illustrating an exemplary process of CT scanning according to some embodiments of the present disclosure;

FIG. 12 is a flowchart illustrating an exemplary process of CT scanning according to some embodiments of the present disclosure;

FIG. 13 is a schematic diagram illustrating an exemplary interactive interface of a CT system according to some embodiments of the present disclosure;

FIG. 14 is a schematic diagram illustrating an exemplary execution process of a scanning instruction of a CT system according to some embodiments of the present disclosure;

FIG. 15 is a flowchart illustrating an exemplary process of CT scanning according to some embodiments of the present disclosure;

FIG. 16 is a flowchart illustrating an exemplary process of CT scanning on a myocardium according to some embodiments of the present disclosure;

FIG. 17 is an exemplary high-energy coronary computed tomography angiography (CTA) image according to some embodiments of the present disclosure;

FIG. 18 is a diagram illustrating an exemplary myocardial blood flow pseudo-color image according to some embodiments of the present disclosure;

FIG. 19 is an exemplary virtual dual-energy image according to some embodiments of the present disclosure;

FIG. 20 is a diagram illustrating an exemplary extracellular volume fraction (ECV) image according to some embodiments of the present disclosure;

FIG. 21 is a flowchart illustrating an exemplary process of CT scanning on a bone tissue according to some embodiments of the present disclosure; and

FIG. 22 is a schematic diagram illustrating an exemplary structure of a processing device according to some embodiments of the present disclosure.

## DETAILED DESCRIPTION

In the following detailed description, numerous specific details are set forth by way of examples to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, to avoid unnecessarily obscuring aspects of the present disclosure. Various modifications to the disclosed embodiments will be readily apparent to those skilled in the art, and the general principles defined herein may be applied to other embodiments and applications without departing from the scope of the present disclosure. Thus, the present disclosure is not limited to the embodiments shown, but is to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including" when used in this specification, specify the presence of stated features, integers, steps, operations, elements, and/or components, but do not preclude the presence or addition of one or more other features, integers, steps, operations, elements, components, and/or groups thereof.

It will be understood that the terms "system," "engine," "unit," "module," and/or "block" used herein are one method to distinguish different components, elements, parts, sections or assemblies of different levels in ascending order. However, the terms may be displaced by other expressions if they achieve the same purpose.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

CT perfusion imaging provides information regarding tissue and organ blood supply. Conventional non-contrast CT scanning process and conventional contrast-enhanced scanning process acquire data at a single time point. During a CT perfusion imaging process, a time attenuation curve (TAC) of each voxel in a tissue or organ is obtained by continuously scanning in a plurality of phases. The TAC reflects an inflow and outflow process (e.g., a blood perfusion process) of a contrast agent in the tissue. Various perfusion parameters, such as, a cerebral blood flow (CBF), a cerebral blood volume (CBV), a mean transit time (MTT), a time to peak (TTP), and a time to maximum residue function (Tmax), are calculated using different mathematical models. Then, these parameters generate perfusion images used to assess tissue ischemia and guide treatment planning.

Spectrum CT imaging differentiates materials by simultaneously acquiring data using both a high energy level and a low energy level from a same anatomical location. Spectrum CT imaging has been widely researched and applied, for example, in the identification and quantitative measurement of materials such as stones, gout deposits, tumor homogeneity, fat content, iodine concentration, and iron content. Compared with conventional CT, which uses a single energy level for anatomical identification, the spectrum CT imaging can provide multi-dimensional functional imaging information, offering much comprehensive references for clinical diagnosis.

Based on the principles of the CT perfusion imaging and the spectrum CT imaging, the combination of spectrum CT and CT perfusion scanning has emerged to obtain comprehensive and detailed diagnostic information. However, the existing combination of spectrum CT and CT perfusion scanning mainly adopts a multi-phase scanning approach. For example, the entire scanning process is divided into three phases: in the first phase and the second phase, perfusion images are acquired using a CT perfusion scan mode, while in the third phase, spectrum images are obtained using a spectrum CT scan mode. Essentially, the CT perfusion scan mode and the spectrum CT scan mode are performed separately in different phases, which results in that data of only one scan mode is obtained during a certain phase and data of both two scan modes is failed to obtain during a same phase. This reduces the amount of information that the images can convey and the images are unfavorable for clinical diagnosis. For example, in a bone CT perfusion scanning, due to a relatively weak iodine enhancement and a small size of blood vessels, an enhanced CT values of conventional perfusion contrast agents are often indistinguishable from those of the bone, thus it is difficult to accurately identify input CT values of the contrast agent during the CT perfusion scanning. As another example, in a myocardial CT perfusion scanning, traditional methods cannot achieve a one-stop vascular assessment. Although the current one-stop perfusion and angiographic imaging allows for simultaneous evaluation of anatomical and functional imaging, in actual clinical practice, capturing the coronary imaging during myocardial perfusion is often challenging, resulting in inconsistent image quality of coronary vessels.

The present disclosure provides a method, device, and system for CT scanning. The method comprises obtaining a scanning instruction for a target object; in response to the scanning instruction, performing a CT perfusion scanning on the target object in the perfusion scanning phase other than the spectrum scanning phase, and performing a spectrum CT scanning on the target object in the spectrum scanning phase. The scanning instruction is configured to instruct to perform a combination of spectrum CT and CT perfusion scanning on the target object. The scanning instruction includes scanning time information of the combination of spectrum CT and CT perfusion scanning. The scanning time information includes a spectrum scanning phase and a perfusion scanning phase, and the spectrum scanning phase is one or more scanning phases of the perfusion scanning phase.

According to embodiments of the present disclosure, by setting a spectrum scanning phase to be one or more scanning phases of the perfusion scanning phase, the spectrum scanning phase is synchronized with one or more scanning phases of the perfusion scanning phase, and both spectrum scanning data and perfusion scanning data are obtained at the same time, which realizes a seamless integration of the spectrum CT scanning and CT perfusion scanning in one scanning process, significantly improves the scanning efficiency while avoids having to abandon a certain scan mode at a certain phase of the scanning process. Within a same scanning cycle, much comprehensive data can be obtained, ensuring the completeness of information in both perfusion images and spectrum images, thereby providing detailed and valuable reference data for clinical diagnosis. For example, in the bone CT perfusion scanning, the method disclosed in the present disclosure can separate calcium and iodine in each spectrum phase to obtain spectral iodine images (also called iodine concentration images). Using the spectral iodine images as perfusion inputs eliminates the influence of bone, thereby avoiding the traditional difficulty in distinguishing between bone CT values and contrast agent-enhanced CT values. Additionally, the perfusion inputs are iodine-enhanced, which is conducive to obtaining an accurate perfusion assessment. As another example, in the myocardial CT perfusion imaging, the method disclosed in the present disclosure enables spectrum CT imaging at each perfusion phase. This allows performing the myocardial CT perfusion imaging based on data generated by spectrum CT imaging using a low energy level (e.g., low kV level), thereby improving vascular imaging quality, and also supports multi-phase cardiac spectrum CT imaging based on spectrum data. Quantitative myocardial perfusion parameters such as myocardial blood flow (MBF) can be used to assess myocardial ischemia, while iodine images can be utilized to observe changes in iodine uptake across different phases, aiding in tumor characterization, such as differentiating benign lesions from malignant lesions. Clinically, this provides more comprehensive functional imaging information to assist in diagnosis. Additionally, the method disclosed in the present disclosure allows customized spectral CT scanning to be performed synchronously without interrupting CT perfusion scanning, and such flexibility provides options and possibilities for combining the two scanning modes, thereby meeting personalized scanning requirements under different clinical needs.

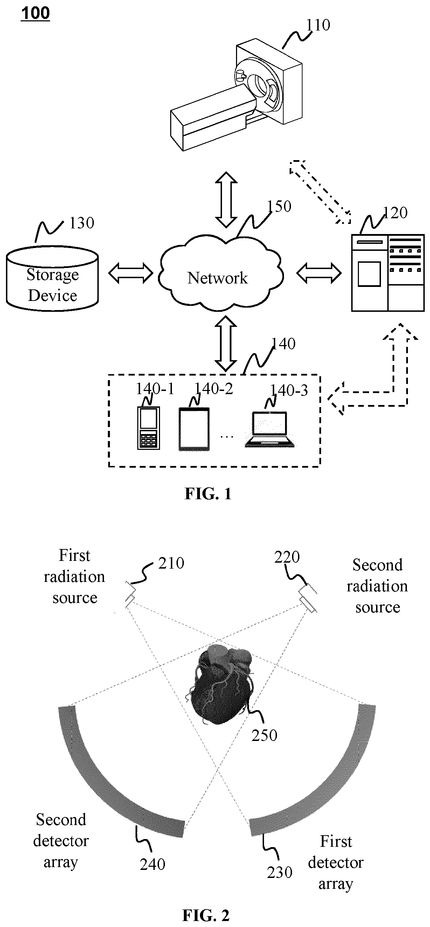

FIG. 1 is a schematic diagram illustrating an exemplary CT system according to some embodiments of the present disclosure. As shown in FIG. 1, the CT system 100 includes an imaging device 110, a processing device 120, a storage device 130, one or more terminals 140, and a network 150. In some embodiments, the imaging device 110, the processing device 120, the storage device 130, and/or the one or more terminals 140 are connected to and/or communicate with each other via a wireless connection, a wired connection, or a combination thereof.

The imaging device 110 may be configured to scan a target object (or a portion of the target object) to acquire medical image data associated with the target object. The target object refers to an object being scanned, such as a biological body, a phantom, etc. The medial image data associated with the target object is configured to generate a computed tomography (CT) image of the target object. The CT image illustrates an internal structure and a health condition of the target object. In some embodiments, the CT image includes a spectrum image and a perfusion image. In some embodiments, the imaging device 110 includes a single-modality scanner and/or a multi-modality scanner. The single-modality scanner includes a CT scanner. The multi-modality scanner includes, for example, a positron emission tomography-computed tomography (PET-CT) scanner, a single-photon emission computed tomography-magnetic resonance imaging (SPECT-MRI) scanner, etc. It should be noted that the imaging device 110 described below is merely provided for illustration purposes, and is not intended to limit the scope of the present disclosure.

Merely by way of example, the imaging device 110 includes a computed tomography scanner and a scanning bed coupled to the computed tomography scanner. The computed tomography scanner forms a scanning cavity, the scanning bed is configured to support the target object, and the scanning bed is movable and capable of moving the target object inside the scanning cavity. The computed tomography scanner includes an X-ray source module and a detector module. The X-ray source module includes one or more X-ray sources for emitting X-rays during a CT scanning process. The detector module includes one or more detector arrays for absorbing the X-rays and generating absorption information of the X-rays. In some embodiments, a functionality, a size, a type, a geometry, a location, a count, and/or an amplitude of the X-ray source module and/or detector module may be determined or varied based on one or more specific conditions. The X-ray source module and the detector module may be designed to surround the target object to form a tunnel type or an open type.

The imaging device 110 is a dual-energy CT scanning device including at least two radiation sources (e.g., two X-ray sources) and two detector arrays. In some embodiments, the imaging device 110 includes two radiation sources and two detector arrays, the two detector arrays corresponding to each of the two radiation sources, respectively. The two radiation sources include a first radiation source and a second radiation source, and the two detector arrays include a first detector array and a second detector array. The first detector array corresponds to the first radiation source, and the second detector array corresponds to the second radiation source. For example, the first detector array detects X-rays emitted by the first radiation source, and the second detector array detects X-rays emitted by the second radiation source. An exemplary structure of the imaging device 110 is described with reference to FIG. 2. FIG. 2 is a schematic diagram illustrating an exemplary imaging device in FIG. 1 according to some embodiments of the present disclosure. As shown in FIG. 2, two radiation sources of the imaging device 110 include a first radiation source 210 and a second radiation source 220. The first radiation source 210 is configured to generate low energy X-rays based on a first voltage, and the second radiation source 220 is configured to generate high energy X-rays based on a second voltage. A voltage value of the first voltage is different from a voltage value of the second voltage. Two detector arrays of the imaging device 110 correspond to each of the two radiation sources, respectively. The two detector arrays are configured to receive X-rays transmitted through a target object 250 and generate scanning data characterizing the X-rays transmitted through the target object 250. The two detector arrays include a first detector array 230 and a second detector array 240. The first detector array 230 corresponds to the first radiation source 210 and is configured to receive low energy X-rays from a region of interest (e.g., a to-be-examined site) of the target object 250 irradiated by the first radiation source 210, and the second detector array 240 corresponds to the second radiation source 220 and is configured to receive high energy X-rays from the region of interest of the target object 250 irradiated by the second radiation source 220. The first radiation source 210 and the first detector array 230 form a first X-ray system that is capable of generating a low energy X-rays beam (e.g., an X-ray beam of 80 kVp); and the second radiation source 220 and the second detector array 240 form a second X-ray system that is capable of generating a high energy X-rays beam (e.g., an X-ray beam of 140 kVp). The two X-ray systems (the first X-ray system and the second X-ray system), are mounted in a same plane at an angle for synchronized scanning.

In some embodiments, the imaging device 110 includes more than two radiation sources and two detector arrays. At least one of the radiation sources is configured to generate low energy X-rays based on a first voltage, and at least one of the radiation sources is configured to generate high energy X-rays based on a second voltage. The radiation source configured to generate low energy X-rays is different from the radiation source configured to generate high energy X-rays. For example, a plurality of first radiation sources are configured to generate low energy X-rays, and one second radiation source is configured to generate high energy X-rays. As another example, a plurality of first radiation sources are configured to generate high energy X-rays, and one second radiation source is configured to generate low energy X-rays. As another example, a plurality of first radiation sources are configured to generate low energy X-rays, and a plurality of second radiation sources are configured to generate high energy X-rays. As another example, a plurality of first radiation sources are configured to generate high energy X-rays, and a plurality of second radiation sources are configured to generate low energy X-rays. One of the two detector arrays corresponds to a radiation source that generates low energy X-rays, and the other of the two detector arrays corresponds to a radiation source that generates high energy X-rays. For example, the first detector array corresponds to one or more first radiation sources, and the second detector array corresponds to one or more second radiation sources.

In some embodiments, the first radiation source and the second radiation source may radiate X-rays at the same time, and the first detector array and the second detector array may receive X-rays at the same time and generate scanning data, thereby avoiding a switching between the first radiation source and the second radiation source, and avoiding large time intervals during scanning.

The processing device 120 is configured to process information and/or data relating to the CT system 100 to perform one or more functions described in the present disclosure. For example, the processing device 120 may be configured to obtain a scanning instruction for a target object. The scanning instruction is configured to perform a combination of spectrum CT and CT perfusion scanning on the target object. The scanning instruction includes scanning time information of the combination of spectrum CT and CT perfusion scanning. The scanning time information includes a spectrum scanning phase and a perfusion scanning phase. The spectrum scanning phase is one or more scanning phases of the perfusion scanning phase. The processing device 120 may, in response to the scanning instruction, perform a CT perfusion scanning on the target object by controlling the second radiation X-ray source to emit single-energy rays based on the single-energy voltage on the target object in the perfusion scanning phase other than the spectrum scanning phase, and perform a spectrum CT scanning on the target object by controlling the first radiation X-ray source to emit dual-energy rays based on the dual-energy voltage on the target object in the spectrum scanning phase. The processing device 120 may generate a perfusion analysis result of the ROI based on the single-energy scanning data and the dual-energy scanning data.

In some embodiments, the processing device 120 is a single server or a server group. The server group is centralized or distributed. In some embodiments, the processing device 120 is local or remote from the CT system 100. In some embodiments, the processing device 120 is implemented on a cloud platform. In some embodiments, the processing device 120 or a portion of the processing device 120 is integrated into the imaging device 110 and/or the one or more terminals 140. It should be noted that the processing device 120 in the present disclosure includes one or a plurality of processors. Thus, operations and/or method steps that are performed by one processor may also be jointly or separately performed by the plurality of processors.

The storage device 130 is configured to store data, instructions, and/or any other information. In some embodiments, the storage device 130 may be configured to store data obtained from the imaging device 110, the processing device 120, and/or the one or more terminals 140. In some embodiments, the storage device 130 may be configured to store data and/or instructions that the processing device 120 may execute or use to perform exemplary methods described in the present disclosure. In some embodiments, the storage device 130 includes a mass storage device, a removable storage device, a volatile read-and-write memory, a read-only memory (ROM), or the like, or a combination thereof. In some embodiments, the storage device 130 is implemented on a cloud platform. In some embodiments, the storage device 130 is a portion of the imaging device 110, the processing device 120, and/or the one or more terminals 140.

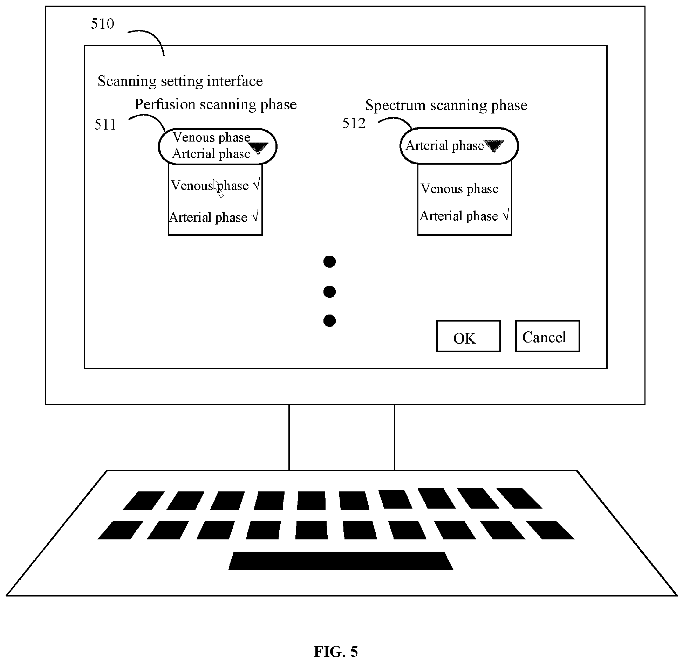

The one or more terminals 140 are configured to enable a user interaction between a user and the CT system 100. The one or more terminals 140 include an interactive interface (e.g., interactive interfaces shown in FIG. 5, FIG. 12, and FIG. 13) for user interaction, through which the user performs interactive operations such as a patient information management, a scan protocol and sequence management, a real-time parameter adjustment, and a system status monitoring. In some embodiments, the one or more terminals 140 may be connected to and/or communicate with the imaging device 110, the processing device 120, and/or the storage device 130. In some embodiments, the one or more terminals 140 may include a mobile device 140-1, a tablet computer 140-2, a laptop computer 140-3, or the like, or a combination thereof. In some embodiments, the one or more terminals 140 may be a portion of the processing device 120 and/or the imaging device 110.

The network 150 may include any suitable network that can facilitate the exchange of information and/or data for the CT system 100. In some embodiments, one or more components of the CT system 100 (e.g., the imaging device 110, the processing device 120, the storage device 130, the one or more terminals 140, etc.) may communicate information and/or data with one or more other components of the CT system 100 via the network 150.

It should be noted that the above description is intended to be illustrative, and not to limit the scope of the present disclosure. Various alternatives, modifications, and variations will be apparent to those skilled in the art. The features, structures, methods, and characteristics of the exemplary embodiments described herein may be combined in various ways to obtain additional and/or alternative exemplary embodiments. In some embodiments, the CT system 100 may include one or more additional components, and/or one or more components described above may be omitted. Additionally, two or more components of the CT system 100 may be integrated into a single component. For example, the processing device 120 may be integrated into the imaging device 110. As another example, a component of the CT system 100 may be replaced by another component that can implement the functions of the component. However, those variations and modifications do not depart from the scope of the present disclosure.

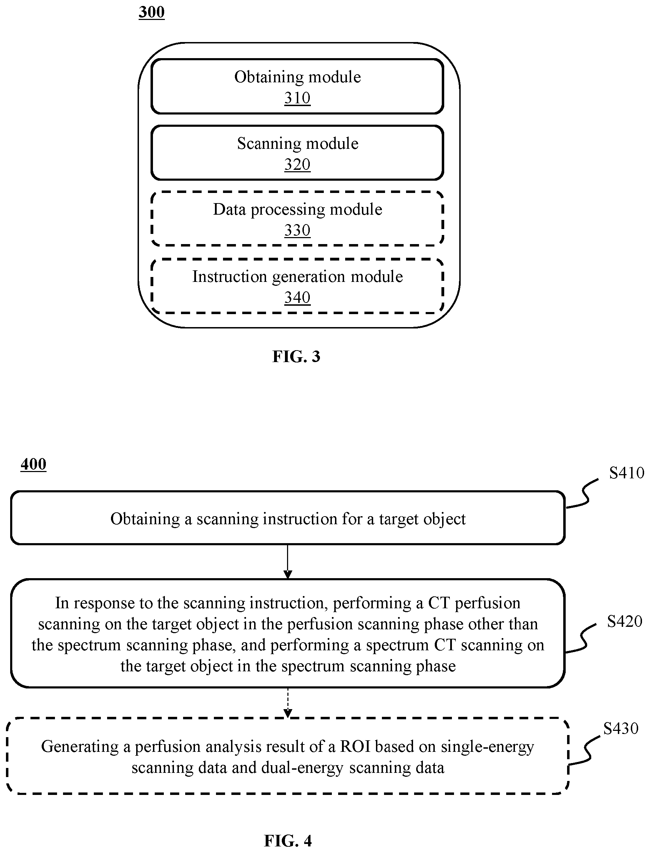

FIG. 3 is a schematic diagram illustrating an exemplary CT system according to some embodiments of the present disclosure. As shown in FIG. 3, a CT system 300 includes an obtaining module 310 and a scanning module 320. In some embodiments, the modules of the CT system 300 are implemented by the processing device 120.

The obtaining module 310 is configured to obtain a scanning instruction for a target object. The scanning instruction is configured to perform a combination of spectrum CT and CT perfusion scanning on the target object. The scanning instruction includes scanning time information of the combination of spectrum CT and CT perfusion scanning. The scanning time information includes a spectrum scanning phase and a perfusion scanning phase. The spectrum scanning phase is one or more scanning phases of the perfusion scanning phase. More descriptions of obtaining the scanning instruction for the target object can be found elsewhere in the present disclosure (e.g., an operation S410 and the descriptions thereof).

The scanning module 320 is configured to, in response to the scanning instruction, performing a CT perfusion scanning on the target object in the perfusion scanning phase other than the spectrum scanning phase, and performing a spectrum CT scanning on the target object in the spectrum scanning phase.

The scanning instruction further includes energy information of the combination of spectrum CT and CT perfusion scanning, the energy information includes a single-energy voltage and a dual-energy voltage. The scanning module 320 is configured to, obtain, using a perfusion scanning mode, single-energy scanning data of a region of interest (ROI) of the target object by emitting single-energy rays based on a single-energy voltage on the target object in the perfusion scanning phase other than the spectrum scanning phase, and obtain dual-energy scanning data of the ROI of the target object by emitting dual-energy rays based on a dual-energy voltage on the target object in the spectrum scanning phase. More descriptions of performing of the CT perfusion scanning and the spectrum CT scanning can be found elsewhere in the present disclosure (e.g., operations S320, S1220, S1510, S1620, S2120, and the descriptions thereof).

In some embodiments, the CT system 300 further includes a data processing module 330. The data processing module 330 is configured to generate a perfusion analysis result of the ROI based on the single-energy scanning data and the dual-energy scanning data. More descriptions of the generation of the perfusion analysis result can be found elsewhere in the present disclosure (e.g., operations S330, S1120, S1420-S1430, and the descriptions thereof).

In some embodiments, the CT system 300 further includes an instruction generation module 340. The instruction generation module 41 is configured to generate, in response to an operation of a user on an interactive interface of the CT system 300, the scanning instruction for the target object (e.g., a scanning instruction for the target object). More descriptions of the generation of the scanning instruction can be found elsewhere in the present disclosure (e.g., operations S1210, S1610, S2110, and the descriptions thereof).

It should be noted that the above descriptions of the CT system 300 are provided for the purposes of illustration and are not intended to limit the scope of the present disclosure. For persons having ordinary skills in the art, various modifications and changes in the forms and details of the application of the above system may occur without departing from the principles of the present disclosure. In some embodiments, the CT system 300 may include one or more other modules, and/or one or more modules described above may be omitted. Additionally, two or more modules may be integrated into a single module, and/or a module may be divided into two or more units. However, those variations and modifications also fall within the scope of the present disclosure.

FIG. 4 is a flowchart illustrating an exemplary process of CT scanning according to some embodiments of the present disclosure. In some embodiments, a process 400 may be executed by the CT system 100 and/or the CT system 200. For example, the process 400 may be implemented as a set of instructions (e.g., an application) stored in a storage device (e.g., the storage device 130 illustrated in FIG. 1). In some embodiments, the processing device 120 of the CT system 100 and/or one or more modules of the CT system 300 may execute the set of instructions and may accordingly be directed to execute the process 400. Unless otherwise specified, the following description takes the CT system 100 as an example.

Step S410, a scanning instruction for a target object is obtained. In some embodiments, step S410 is performed by the obtaining module 310.

The target object refers to an object being scanned. The scanning instruction refers to an instruction for performing a combination of spectrum CT and CT perfusion scanning on the target object.

For example, the scanning instruction may be a computer instruction input by a user that instructs to perform scanning on some organ or tissue of a patient. The scanning instruction includes scanning time information of the combination of spectrum CT and CT perfusion scanning. In some embodiments, the scanning instruction further includes energy information of the combination of spectrum CT and CT perfusion scanning. The combination of spectrum CT and CT perfusion scanning refers to a spectrum CT scanning and a CT perfusion scanning being performed simultaneously on a same CT system.

The energy information refers to information reflecting the energy of the rays, including a single-energy voltage and a dual-energy voltage (or a high-and-low energy voltage). The dual-energy voltage includes a first voltage and a second voltage, which have different voltage values. The second voltage is configured to generate high-energy rays and the first voltage is configured to generate low-energy rays. The high-and-first voltage is the voltage required for the spectrum CT scanning. One of a second voltage and a first voltage is the voltage required for the CT perfusion scanning. The single-energy voltage is one of a second voltage and a first voltage of the dual-energy voltage. For example, if the first voltage is 80 kV and the second voltage is 140 kV, the single-energy voltage is either 80 kV or 140 kV. Unless otherwise specified, the following description takes the voltage required for CT perfusion scanning as the first voltage. It should be appreciated that second voltage is typically in a range of 130 kV to 150 kV, and the first voltage is typically in a range of 70 kV to 100 kV.

The scanning time information includes a spectrum scanning phase and a perfusion scanning phase. The term "scanning phase" refers to a starting point of a scanning process or a period of the scanning process. For example, the scanning phase includes an arterial phase, a venous phase, and a delayed phase within a single scanning process. As another example, the scanning phase refers to one or more starting points within the same scanning process. The scanning time information refers to information reflecting a scanning phase, including a spectrum scanning phase and a perfusion scanning phase. The spectrum scanning phase refers to a scanning phase for performing the spectrum CT scanning, and the perfusion scanning phase refers to a scanning phase for performing the CT perfusion scanning. The spectrum scanning phase is one or more scanning phases of the perfusion scanning phase. In some embodiments, the spectrum scanning phase is a portion of the perfusion scanning phase, or the spectrum scanning phase is identical to the perfusion scanning phase. When the spectrum scanning phase is a portion of the perfusion scanning phase, the spectrum scanning phase includes a portion of the perfusion scanning phase. When the spectrum scanning phase is identical to the perfusion scanning phase, the scanning phases in the spectrum scanning phase are identical to scanning phases of the perfusion scanning phase. For example, the perfusion scanning phase includes at least one of a venous phase, an arterial phase, or a delayed phase, and the spectrum scanning phase is at least one of the venous phase and/or the arterial phase and/or the delayed phase included in the perfusion scanning phase. As another example, if the perfusion scanning phase includes both a venous phase and an arterial phase, and the spectrum scanning phase is a portion of the perfusion scanning phase rather than identical to the perfusion scanning phase, thus, the spectrum scanning phase includes either the venous phase or the arterial phase. As another example, if the perfusion scanning phase includes both a venous phase and an arterial phase, the spectrum scanning phase is identical to the perfusion scanning phase, and the spectrum scanning phase includes both the venous phase and the arterial phase.

In some embodiments, each scanning phase includes one or more sampling times. In some embodiments, the scanning phase includes at least one first sampling time and at least one second sampling time. The first sampling time refers to a sampling period in the perfusion scanning phase. For example, the first sampling time is a time period between a starting time point of a scanning phase of the perfusion scanning phase and an ending time point of the scanning phase of the perfusion scanning phase. The second sampling time refers to a sampling period in the spectrum scanning phase. For example, the second sampling time is a time period between a starting time point of a scanning phase of the spectrum scanning phase and an ending time point of the scanning phase of the spectrum scanning phase. Based on emission times of high-energy rays and low-energy rays in a same sampling period, when the high-energy rays and the low-energy rays in the same sampling period are emitted sequentially (e.g., the low-energy rays are emitted first, then the high-energy rays are emitted), each second sampling time corresponds to a first sampling time. For example, a single sampling period in the spectrum scanning phase is identical to a single sampling period in the perfusion scanning phase. When the high-energy rays and the low-energy rays are emitted at the same time in the same sampling period, the at least one second sampling time is one or more of the at least one first sampling time. For example, the single sampling period in the spectrum scanning phase includes one or more sampling periods in the perfusion scanning phase. The energy information corresponding to the first sampling time is a first single-energy voltage, and the energy information corresponding to the second sampling time is a second single-energy voltage. In some embodiments, the first single-energy voltage is the voltage (e.g., one of a second voltage and a first voltage of a dual-energy voltage) required for the CT perfusion scanning, and the second single-energy voltage is the other of the second voltage and the first voltage of the dual-energy voltage. Merely by way of example, assuming that the high-energy rays and the low-energy rays are emitted sequentially, and a CT perfusion scanning includes 20 scanning phases. The last 2 scanning phases of the 20 scanning phases are designated as the spectrum scanning phases. In this way, in the first 18 scanning phases, low-energy rays are emitted, and high-energy rays are emitted only during the spectrum scanning phases. When the high-energy rays are emitted in the spectrum scanning phases, the low-energy rays are not discontinued. That is, both the high-energy rays and the low-energy rays are emitted simultaneously during the spectrum scanning phases.

In some embodiments, the energy information and the scanning time information of the combination of spectrum CT and CT perfusion scanning may be manually set by the user in real time or determined based on empirical values. For example, the processing device 120 establishes correlations between energy information and scanning time information of the various sites when the combination of spectrum CT and CT perfusion scanning is performed, and stores the correlations. After selecting a target object for the combination of spectrum CT and CT perfusion scanning during an examination phase, the processing device 120 obtains energy information and scanning time information of the target object based on the correlation.

In some embodiments, the scanning instruction is determined based on the energy information and the scanning time information of the combination of spectrum CT and CT perfusion scanning. Specifically, the processing device 120 receives a first setup operation of a perfusion scanning phase and a spectrum scanning phase for a combination of spectrum CT and CT perfusion scanning on a target object and a second setup operation for a second voltage and a first voltage, and generates energy information and scanning time information. The processing device 120, in response to a confirmation operation of performing the combination of spectrum CT and CT perfusion scanning on the target object, generates a scanning instruction based on the energy information and the scanning time information.

Merely by way of example, FIG. 5 is a schematic diagram illustrating an exemplary interactive interface of a CT system according to some embodiments of the present disclosure. As shown in FIG. 5, the interactive interface is a scanning setting interface 510, which displays a configuration option 511 for a perfusion scanning phase and a configuration option 512 for a spectrum scanning phase. Both the configuration option 511 and the configuration 512 include an arterial phase and a venous phase. Assuming the perfusion scanning phase selected in the configuration option 511 are the arterial phase and the venous phase, the spectrum scanning phase in the configuration option 512 may be set to the arterial phase and/or the venous phase. As shown in FIG. 5, if the arterial phase is selected in the configuration option 512, it may be determined that spectrum CT scanning is performed during the arterial phase, while perfusion scanning is performed during both the arterial phase and the venous phase. In other words, dual-energy scanning (e.g., scanning with both high energy and low energy) is performed during the arterial phase, while single-energy scanning (e.g., scanning with one of high energy or low energy ) is performed during the venous phase. In some embodiments, the scanning setting interface may further include configuration options for other parameters, such as voltage values for dual-energy scanning, a scanning duration, a sampling time, and single-energy scanning data used in the perfusion scanning phase other than the spectrum scanning phase. Users may configure relevant options in the scanning setting interface based on practical application needs, and the embodiments of the present disclosure place no limitations in this regard.

In some embodiments, the scanning instruction may also be obtained through other means, such as retrieving a pre-configured scanning instruction from a storage device (e.g., the storage device 130).

Merely by way of example, in a CT perfusion scanning workflow, the process begins with registering the patient undergoing the CT perfusion scanning, followed by acquiring the initial CT scanning images of the patient, which are used as reference images. For example, low-dose scanning may be performed to obtain the reference images, which serve to guide scanning positioning and planning. Subsequently, based on the reference images, a positioning slice and a broad coverage range for the CT perfusion scanning are planned. For example, key anatomical landmarks, e.g., the location of organs or lesions, may be selected to ensure that a scanning range covers all regions that need to be examined. For a combination of spectrum CT and CT perfusion scanning (e.g., myocardial perfusion), a configuration of whether to perform a spectrum data acquisition for one or more phases of each scanning stage is made. For example, the spectrum data acquisition may be performed in all phases during a computed tomography angiography (CTA) stage. Once the spectrum data acquisition is configured, parameters such as high and low energy voltages (kV), a contrast agent dosage, and a sampling time (e.g., sampling interval) may be set via the corresponding setting interface. Further, it may be also necessary to configure reconstruction parameters for the scanning sequence. Specifically, individual sequences may be set for CTA, computed tomography perfusion (CTP), dual-energy computed tomography perfusion (DECTP), and dual-energy computed tomography perfusion (DE). The CTA and CTP sequences correspond to dedicated angiographic and perfusion sequences, respectively, while the DE and DECTP sequences correspond to spectrum CT imaging sequences and spectrum-based perfusion sequences. Once the relevant parameters have been configured and confirmed, a scanning task may be initiated. After the scanning process is completed, the acquired scanning data may be loaded for subsequent image review, image reconstruction, and post-processing analysis, such as vascular analysis, spectrum analysis, and perfusion analysis.

In some embodiments, for a combination of spectrum CT and CT perfusion scanning, when certain phases of the CT perfusion scanning are configured as spectrum scanning phases (e.g., the spectrum scanning phase is a portion of the perfusion scanning phase), a DE sequence is reconstructed, and the reconstruction of the DE sequence may support conventional spectrum image types, such as virtual monoenergetic images (VMIs) at different kilovolt peak (kVp) levels (e.g., different energy levels), iodine images, and optimal contrast-to-noise ratio (CNR) images. When all phases of the CT perfusion scanning are configured as spectrum scanning phases (e.g., the spectrum scanning phases are fully identical to the perfusion scanning phases), a DECTP sequence is reconstructed, and the reconstruction of the DECTP sequence may support conventional spectrum image types, such as virtual monoenergetic images (VMIs) at different kilovolt peak (kVp) levels, iodine images, and optimal contrast-to-noise ratio (CNR) images. These spectrum image types may serve as subsequent inputs for each phase of perfusion analysis.

In some embodiments, the processing device 120 generates a scanning instruction for a target object in response to an operation of a user on the interactive interface of the CT system (e.g., an interactive interface in the terminal 140). For example, the scanning instruction for the target object may be a scanning instruction for a to-be-examined site of the target object. The scanning instruction may indicate a scanning mode specified by the user, which includes a dual-energy scanning or a combination of single-energy scanning and dual-energy scanning. The dual-energy scanning refers to scanning performed using both a high voltage and a low voltage, whereas the single-energy scanning refers to scanning performed using only a low voltage. More details on the generation of the scanning instruction can be found in steps S1210 and S2110.

Step S420, in response to the scanning instruction, a CT perfusion scanning on the target object in the perfusion scanning phase other than the spectrum scanning phase is performed, and a spectrum CT scanning on the target object in the spectrum scanning phase is performed. In some embodiments, step S420 is performed by the scanning module 320.

The processing device 120 performs the CT perfusion scanning to obtain single-energy scanning data of a region of interest (ROI) of the target object, and performs the spectrum CT scanning to obtain dual-energy scanning data of the ROI of the target object. The processing device 120 obtains, using a perfusion scanning mode, the single-energy scanning data of a region of interest (ROI) of the target object, by emitting single-energy rays based on a single-energy voltage on the target object in the perfusion scanning phase other than the spectrum scanning phase, and obtains the dual-energy scanning data of the ROI of the target object by emitting dual-energy rays based on a dual-energy voltage on the target object in the spectrum scanning phase.

The processing device 120, based on energy information and the scanning timing information of the combination of spectrum CT and CT perfusion scanning in the scanning instruction, emits the dual-energy rays using the dual-energy voltage in the spectrum scanning phase and the perfusion scanning mode. For example, a spectrum CT scanning is performed during the spectrum scanning phase. In this manner, the dual-energy scanning data may be obtained by performing the spectrum CT scanning when a contrast agent is being injected or after the contrast agent is injected. A scanning process using a second voltage and a first voltage is referred to as the dual-energy scanning.

In some embodiments, when the single-energy voltage is a low voltage, the corresponding single-energy rays are low-energy rays, and low-energy scanning data of the scanning data is used for image reconstruction to generate a perfusion image. When the single-energy voltage is a high voltage, the corresponding single-energy rays are high-energy rays, and high-energy scanning data of the scanning data is used for image reconstruction to generate a perfusion image. The scanning process performed using a single-energy voltage is referred to as a single-energy scanning.

In some embodiments, in response to the scanning instruction, the processing device 120, using the perfusion scanning mode, emits first single-energy rays based on a first single-energy voltage to the target object within at least one first sampling time and emits second single-energy rays based on a second single-energy voltage within at least one second sampling time, to obtain first scanning data corresponding to the first single-energy rays and second scanning data corresponding to the second single-energy rays for the ROI of the target object. Since at least one first sampling time corresponds to a sampling time point in the perfusion scanning phase, and at least one second sampling time corresponds to a sampling time point in the spectrum scanning phase, when the spectrum scanning phase is a portion of the perfusion scanning phase (and the spectrum scanning phase is not identical to the perfusion scanning phase), the single-energy scanning data includes scanning data of the perfusion scanning phase other than the spectrum scanning phase in the first scanning data, referred to as first single-energy scanning data. The dual-energy scanning data includes scanning data of the spectrum scanning phase in the first scanning data and second scanning data. The second scanning data is also referred to as second single-energy scanning data, and the scanning data of the spectrum scanning phase in the first scanning data is referred to as fourth single-energy scanning data.

In some embodiments, the processing device 120 utilizes the dual-energy scanning data to obtain the single-energy scanning data required for the spectrum scanning phase when the spectrum scanning phase is identical to the perfusion scanning phase. Merely by way of example, the processing device 120, based on dual-energy CT scanning parameters in the scanning instruction, which includes a low-energy voltage and a high-energy voltage (e.g., the low-energy voltage is 80 kV and the high-energy voltage is 140 kV), injects an iodine contrast agent during the scanning process, and obtains the dual-energy scanning data (including low-energy scanning data and high-energy scanning data) by acquiring a dynamic distribution of the contrast agent in a bloodstream of the target object by successive scans. Subsequently, the processing device 120 may select one of the dual-energy scanning data to construct a perfusion image based on actual needs.

In a CT perfusion scanning, the target object is injected with a contrast agent. In some embodiments, in response to the target object being injected with the contrast agent, the processing device 120 controls the CT system to execute the scanning instruction to obtain the scanning data. An execution process of the scanning instructions is divided into a plurality of phases based on the dose of the contrast agent in the ROI (e.g., blood vessels, heart, bone tissue, etc.) of the target object, and at least one phase of the plurality of phases is used to perform the dual-energy scanning to acquire the dual-energy scanning data. In the dual-energy scanning, the processing device 120 controls two radiation sources (e.g., the first radiation source 210 and the second radiation source 220) to execute the scanning instruction and two detector arrays (e.g., the first detector array 230 and the second detector array 240) to acquire the scanning data.

In some embodiments, at least one phase of the plurality of phases performs a single-energy scanning, and at least one phase of the plurality of phases performs a dual-energy scanning. The scanning data includes the single-energy scanning data and the dual-energy scanning data. The single-energy scanning data is acquired through the single-energy scanning, and the dual-energy scanning data is acquired through the dual-energy scanning. In the single-energy scanning, the processing device 120 controls a radiation source (e.g., the first radiation source 210 or the second radiation source 220) to execute the scanning instruction and a detector array corresponding to the radiation source (e.g., the first detector array 230 or the second detector array 240) to acquire the scanning data.

In some embodiments, the processing device 120 perform operation S430 after operation S420.

Step S430, a perfusion analysis result of the ROI is generated based on the single-energy scanning data and the dual-energy scanning data. In some embodiments, step S430 is performed by the data processing module 330.

The perfusion analysis result refers to a medical examination result obtained based on a perfusion scanning technique. For example, the perfusion analysis result includes a blood supply of tissues and organs. The processing device 120 may generate the perfusion analysis result in different ways based on a relationship between the spectrum scanning phase and the perfusion scanning phase.

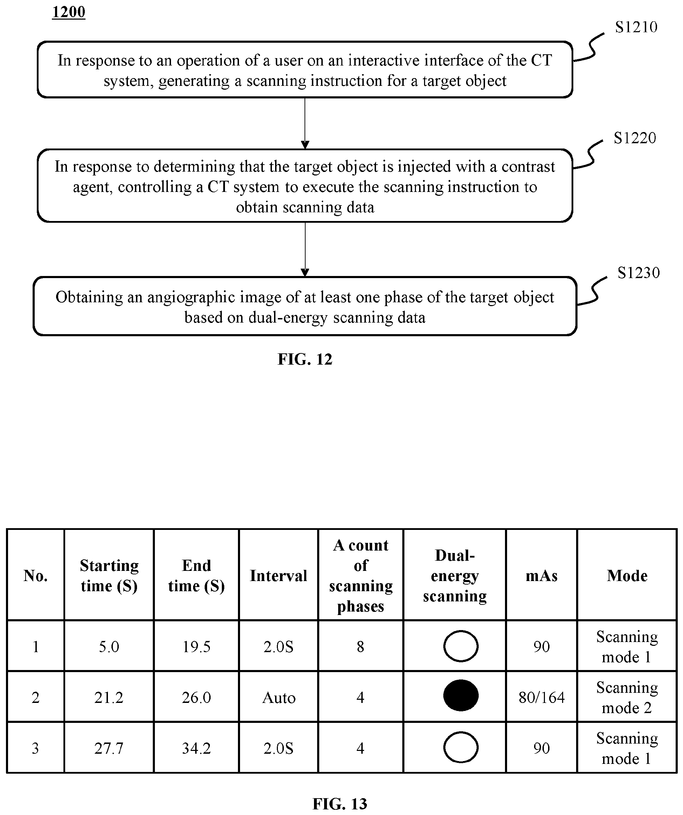

In some embodiments, the processing device 120 selects data of at least one phase of the scanning data for reconstruction to obtain a target image of the target object. Specifically, based on a reconstruction mode, the processing device 120 selects the dual-energy scanning data and/or the single-energy scanning data of at least one phase for reconstruction to obtain the target image of the target object. The reconstruction mode is obtained based on an operation of the user on the interactive interface of the CT system. The reconstruction mode includes at least one of an angiography reconstruction mode, a perfusion imaging reconstruction mode, a spectrum reconstruction mode, or the like. The target image corresponds to the reconstruction mode. For example, when the reconstruction mode is the angiography reconstruction mode, the target image is an angiographic image of at least one phase of the target object. As another example, when the reconstruction mode is the perfusion imaging reconstruction mode, the target image is a perfusion image of at least one phase of the target object. In some embodiments, the target image corresponds to the target object. For example, when the target object is the heart of a patient, the target image includes at least one of a high-energy myocardial vascular image, a low-energy myocardial vascular image, a virtual single-energy image, a spectrum curve, a perfusion image, or the like. As another example, when the target object is a bone tissue, the target image includes iodine concentration images of a plurality of phases. After acquiring the target image, the processing device 120 displays the target image to the user on the interactive interface. More content on obtaining the target image by reconstructing the scanning data can be found in FIG. 12, FIG. 15, FIG. 16, and FIG. 21.

In some embodiments, when the spectrum scanning phase is a portion of the perfusion scanning phase (and the spectrum scanning phase is not identical to the perfusion scanning phase), the processing device 120 generates a perfusion image of the ROI based on the first single-energy scanning data in the perfusion scanning phase other than the spectrum scanning phase and the second single-energy scanning data of the dual-energy scanning data. The processing device 120 generates a spectrum image of the ROI based on the dual-energy scanning data. The processing device 120 obtains the perfusion analysis result based on the spectrum image and/or the perfusion image.

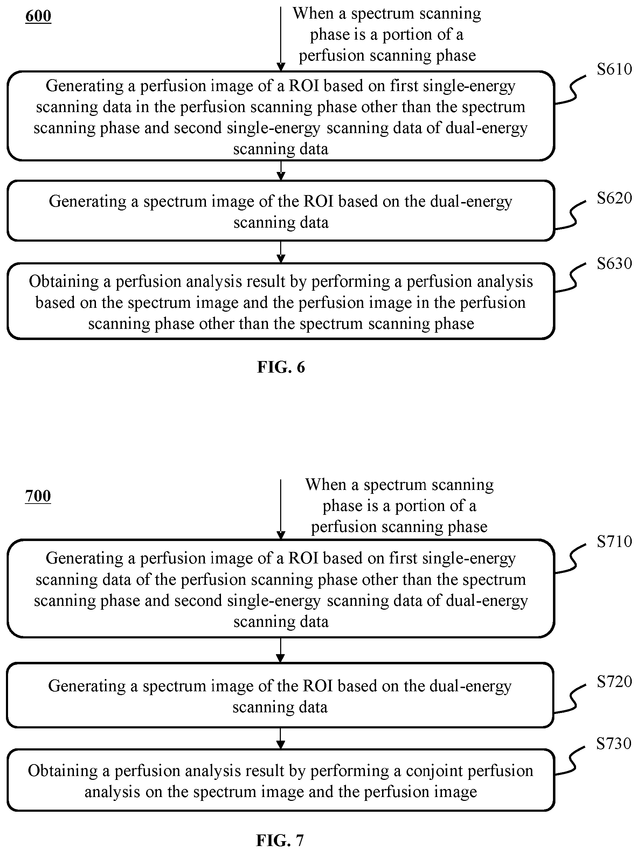

The processing device 120 obtains the perfusion analysis result by performing a perfusion analysis based on the spectrum image and the perfusion image. Alternatively, the processing device 120 obtains the perfusion analysis result by performing a conjoint perfusion analysis on the spectrum image and the perfusion image. More content on obtaining the perfusion analysis result when the spectrum scanning phase is a portion of the perfusion scanning phase can be found in FIG. 6 and FIG. 7.

In some embodiments, when the spectrum scanning phase is identical to the perfusion scanning phase, the processing device 120 generates a medical image (e.g., one or more of a spectrum image, a perfusion image, and a spectrum-perfusion image) based on the dual-energy scanning data and/or the single-energy scanning data, and obtains the perfusion analysis result based on the medical image.

In some embodiments, when the spectrum scanning phase is identical to the perfusion scanning phase, the processing device 120 may obtain the fourth single-energy scanning data of the spectrum scanning phase based on the dual-energy scanning data and generate the perfusion image of the ROI based on the fourth single-energy scanning data. Additionally or alternatively, the processing device 120 may generate the spectrum image of the ROI based on the dual-energy scanning data, and obtain the perfusion analysis result based on the spectrum image and/or the perfusion image.

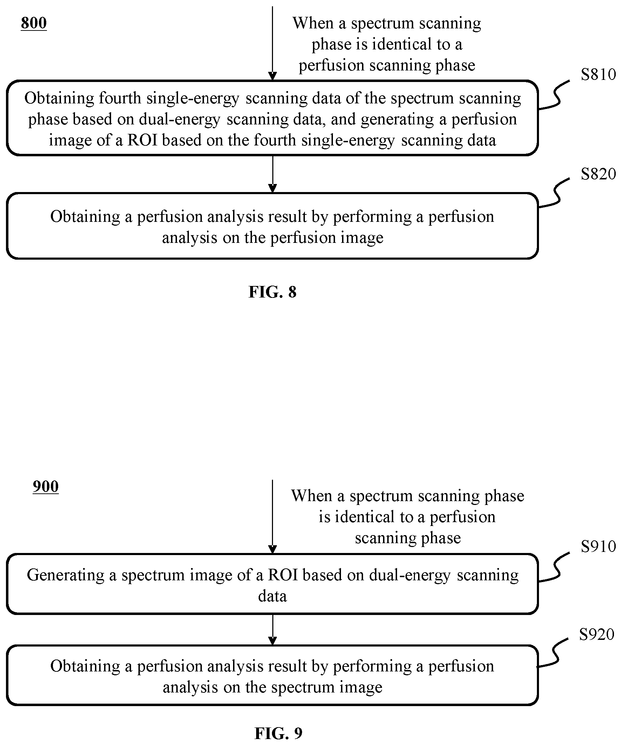

The processing device 120 obtains the perfusion analysis result by performing the perfusion analysis on the spectrum image or the perfusion image. Alternatively, the processing device 120 obtains the perfusion analysis result by performing the conjoint perfusion analysis on the spectrum image and the perfusion image. More content on obtaining the perfusion analysis result based on the spectrum image and/or the perfusion image when the spectrum scanning phase is identical to the perfusion scanning phase can be found in FIG. 8, FIG. 9, and FIG. 10.

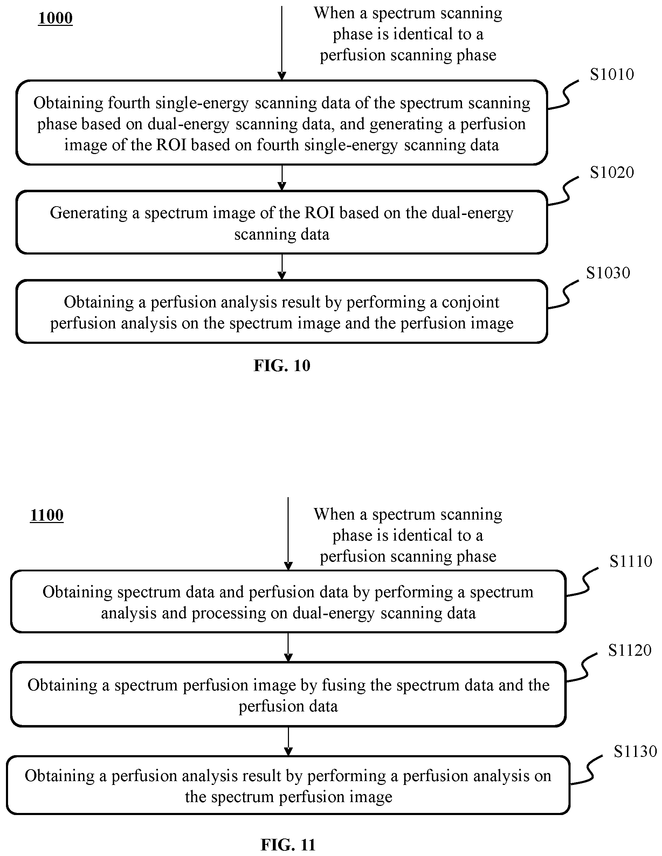

In some embodiments, when the spectrum scanning phase is identical to the perfusion scanning phase, the processing device 120 may obtain spectrum data and perfusion data by performing a spectrum analysis and processing on the dual-energy scanning data, obtain a spectrum perfusion image by fusing the spectrum data and the perfusion data, and generate the perfusion analysis result by performing a perfusion analysis on the spectrum perfusion image. More content on obtaining the perfusion analysis result based on the spectrum perfusion image when the spectrum scanning phase is identical to the perfusion scanning phase can be found in FIG. 11.

In some embodiments, the processing device 120 may output the perfusion analysis result of the ROI by inputting the single-energy scanning data and the dual-energy scanning data into a trained model. The model may include various machine learning models, for example, a CNN and a RNN. The training samples of the model include a plurality of single-energy scanning data samples and a plurality of dual-energy scanning data samples, and the training labels of the model are perfusion analysis results of ROIs corresponding to the plurality of single-energy scanning data samples and the plurality of dual-energy scanning data samples. The direct output of the perfusion analysis result using the model makes the determination of the perfusion analysis result fast and accurate.

In some embodiments of the present disclosure, by using the perfusion scanning mode as a reference scanning mode throughout the scanning process, the single-energy rays are emitted based on the single-energy voltage in the perfusion scanning phase other than the spectrum scanning phase included in the scanning instruction, to obtain the single-energy scanning data required for constructing the perfusion image. Also, the dual-energy rays are emitted based on the dual-energy voltage in the spectrum scanning phase, to obtain the dual-energy scanning data required for constructing the spectrum image and the perfusion image. By setting the spectrum scanning phase to be one or of scanning phases of the perfusion scanning phase, the spectrum scanning phase is synchronized with the one or more scanning phases of the perfusion scanning phase and the spectrum scanning data and the perfusion scanning data are obtained at the same time, which realizes a seamless integration of the spectrum CT scanning and the CT perfusion scanning throughout the scanning process, significantly improving scanning efficiency and avoiding giving up any type of scanning at certain stages. In the same scanning process, the acquired data is comprehensive, which guarantees the comprehensiveness of the perfusion image and the spectrum image, thus providing detailed and valuable reference data for clinical diagnosis.

FIG. 6 is a flowchart illustrating an exemplary process of CT scanning according to some embodiments of the present disclosure. In some embodiments, at least a portion of a process 600 may be performed to achieve at least a portion of step S430 as described in connection with FIG. 4. For example, when a spectrum scanning phase is a portion of a perfusion scanning phase (and is not identical to the perfusion scanning phase), the processing device 120 or the data processing module 330 may generate a perfusion analysis result for a ROI of a target object based on single-energy scanning data and dual-energy scanning data by performing at least a portion of the process 600.

Step S610, a perfusion image of the ROI is generated based on first single-energy scanning data obtained in a perfusion scanning phase other than a spectrum scanning phase and second single-energy scanning data of dual-energy scanning data.

The perfusion image refers to a medical image obtained based on perfusion scanning techniques that reflect the blood supply of tissues, organs, etc. As previously described, in a combination of spectrum CT and CT perfusion scanning, the first single-energy scanning data and the second single-energy scanning data are scanning data of different scanning phases in the perfusion scanning phase. The processing device 120 generates the perfusion image of all perfusion scanning phases by image reconstruction based on the first single-energy scanning data and the second single-energy scanning data. The image reconstruction may be performed using any image reconstruction manners, which is not limited by the present disclosure.

In some embodiments, the processing device 120 generates the perfusion image of the perfusion scanning phase other than the spectrum scanning phase based on the first single-energy scanning data.

Step S620, a spectrum image of the ROI is generated based on the dual-energy scanning data.

The spectrum image refers to a medical image obtained based on a spectrum scanning technique, which is capable of reflecting information about the composition of tissues and organs, etc., such as iodine concentration and calcium content. The spectrum image includes at least one of a virtual single-energy image, an iodine concentration distribution image, or an optimal signal-to-noise ratio image. The spectrum image can well reflect the spectral characteristics of the ROI (e.g., iodine concentration, calcium content, etc.), thereby improving clinical diagnosis. For example, in bone CT perfusion imaging, using iodine images as the perfusion input can easily eliminate the influence of bone, avoiding the traditional problem of poor differentiation between bone CT values and contrast agent-enhanced CT values, while enhancing iodine input and facilitating perfusion assessment. A more detailed description of the spectrum image can be found in step S920 and the related descriptions thereof.

As previously described, in the combination of spectrum CT and CT perfusion scanning, the dual-energy scanning data includes single-energy scanning data of the spectrum scanning phase in the perfusion scanning phase and second single-energy scanning data. The processing device 120 generates a spectrum image corresponding to the spectrum scanning phase by image reconstruction based on the dual-energy scanning data. The image reconstruction may be performed using any image reconstruction manners, which is not limited by the present disclosure.

In some embodiments, the processing device 120 obtains a perfusion image based on the spectrum image. Merely by way of example, assuming that scanning data is a CT value, first scanning data (including the first single-energy scanning data and the fourth single-energy scanning data) is a first CT value, and second scanning data (e.g., the second single-energy scanning data) is a second CT value. The processing device 120 may generate a spectrum image based on the second CT value and the first CT value corresponding to each second sampling time, and obtain new low-energy CT values (e.g., CT values corresponding to the first single-energy rays) of first sampling times corresponding to each second sampling time by performing a CT value conversion calculation on the spectrum image, and designate the new first CT value as third single-energy scanning data. The processing device 120 may generate the perfusion image of the ROI based on a new first CT values of the first sampling times corresponding to each second sampling time, and first CT values of one or more first sampling times that do not correspond to any of the second sampling times.

In some embodiments of the present disclosure, by generating the perfusion image based on the single-energy scanning data and generating the spectrum image based on the dual-energy scanning data when the spectrum scanning phase is a portion of the perfusion scanning phase, images corresponding to the perfusion scanning and the spectrum scanning are obtained based on the scanning data of the CT perfusion scanning and the spectrum CT scanning, respectively, so that the perfusion image and the spectrum image can be obtained by one scanning at the same time, improving the scanning efficiency while guaranteeing the combination of the two types of images.

Step S630, a perfusion analysis result is obtained by performing a perfusion analysis based on the spectrum image and the perfusion image obtained in the perfusion scanning phase other than the spectrum scanning phase.

The perfusion analysis result refers to information that reflects results of a perfusion scanning analysis, such as a perfusion image. Since the spectrum image corresponds to the dual-energy scanning data, the spectrum image includes information required for the perfusion analysis, and the processing device 120 may obtain the perfusion analysis result corresponding to a complete perfusion scanning phase by performing the perfusion analysis on the spectrum image and the perfusion image of the perfusion scanning phase other than the spectrum scanning phase. In this way, the perfusion analysis result can be obtained based on the information obtained from a complete CT perfusion scanning when the spectrum scanning phase is a portion of the perfusion scanning phase, thereby ensuring the completeness of the perfusion analysis result and improving the quality of the perfusion analysis result.

The perfusion analysis may be performed in various ways. For example, the processing device 120 performs the perfusion analysis (e.g., using perfusion analysis software, etc.) on the spectrum image and the perfusion image obtained in the perfusion scanning phase other than the spectrum scanning phase, obtains perfusion parameters (e.g., blood flow rate, blood volume, time to peak, etc.), and obtains the perfusion analysis result regarding the hemodynamics of the tissues of the ROI based on the perfusion parameters. As another example, the processing device 120 obtains the third single-energy scanning data using the spectrum image, and then obtains the perfusion analysis result by performing the perfusion analysis based on the first single-energy scanning data and the third single-energy scanning data.

Merely by way of example, by using the perfusion analysis software, the processing device 120 may outline an ROI (e.g., a to-be-examined site) to be analyzed on the perfusion image. For example, the ROI includes a specific organ, tissue, or lesion area, etc. Definitions of ROIs need to be consistent across all perfusion image sequences for accurate time series analysis. Then, the processing device 120 may generate a time attenuation curve or a time-signal curve based on the perfusion image sequences, which reflects the concentration of the contrast agent in the ROI over time. The processing device 120 may obtain the perfusion analysis result based on the time attenuation curve or the time-signal curve. For example, the perfusion analysis result may include perfusion parameters, and the perfusion parameters include blood flow (BF), blood volume (BV), time to peak (TTP), and mean transit time (MTT), etc. The perfusion analysis result can provide information about hemodynamic properties within the tissue or lesion in the ROI, while the perfusion parameters may be used to assess the functional state of the tissue, for example, tumor angiogenesis, brain tissue ischemia, or infarction.

In some embodiments, the processing device 120 obtains the third single-energy scanning data by performing a scanning data conversion based on the spectrum image. Merely by way of example, if single-energy scanning data is a single-energy CT value, the processing device 120 may use a material decomposition algorithm to decompose the spectrum images at each energy level into iodine and water component images, or other material-specific images of interest, such as calcium or fat. The processing device 120 may select a target energy level (e.g., the energy level corresponding to the desired single energy, for example, the first single-energy voltage), and generate a virtual single-energy image at the target energy level by calculating based on iodine and water component images at high and low energies, or by combining other material-specific images of interest. The processing device 120 may extract the single-energy CT values of the virtual single-energy image and calculate an average CT value for all pixels in the ROI. The average CT value is the single-energy CT value at the selected single-energy level (e.g., the third single-energy scanning data). More content on performing the scanning data conversion to obtain the third single-energy scanning data based on the spectrum image can be found in step S620 and the related descriptions thereof. By obtaining the single-energy scanning data by performing the scanning data conversion on the spectrum image, low-energy scanning data corresponding to the perfusion scanning is separated from the dual-energy scanning data, so that when the spectrum scanning phase is a portion of the perfusion scanning phase, the low-energy scanning data corresponding to a complete perfusion scanning can be obtained, which ensures the completeness of the perfusion image obtained subsequently.

In some embodiments, the processing device 120 obtains the virtual single-energy image by calculation according to the following formula (1):

$$I(E) = I_{\text{low}} \cdot \exp\left(-\mu_{\text{low}}(E) \cdot d\right) + I_{\text{high}} \cdot \exp\left(-\mu_{\text{high}}(E) \cdot d\right) \quad (1),$$

where I(E) denotes an intensity of the virtual single-energy image at energy E, d denotes a path length of the X-rays passing through the target object, and in CT scanning, d is related to a scanning geometrical parameters and an anatomical structure of the target object; $I_{low}$ and $I_{high}$ denote intensity values of the low-energy spectrum images and high-energy spectrum images, respectively, at a same position (e.g., a same path length d), which are obtained from the measurements of a CT scanning device; $\mu_{low}(E)$ and $\mu_{high}(E)$ denote attenuation coefficients of the target object at energy E, corresponding to low-energy X-rays and high-energy X-rays, respectively, and the attenuation coefficients describe absorption capacity of the target object for X-rays and are a function of the energy E, which are obtained by experimental measurements or theoretical calculations and are stored in the spectrum database of a CT scanner; and the exponential terms $\exp(-\mu_{low}(E) \cdot d)$ and $\exp(-\mu_{high}(E) \cdot d)$ denote a proportion of the intensity of low-energy X-rays and high-energy X-rays that have been attenuated after passing through the target object, which are related to a linear attenuation coefficient of the target object and the path length. In some embodiments, different base materials, e.g., iodine, water, calcium, fat, etc., may be calculated by substituting their attenuation coefficients in formula (1).

The processing device 120 obtains the perfusion analysis result by performing the perfusion analysis based on the first single-energy scanning data and the third single-energy scanning data. Specifically, the processing device 120 generates the perfusion image of the ROI based on the first single-energy scanning data and the third single-energy scanning data, and obtains the perfusion analysis result by performing the perfusion analysis on the perfusion image.

FIG. 7 is a flowchart illustrating an exemplary process of CT scanning according to some embodiments of the present disclosure. In some embodiments, at least a portion of a process 700 may be performed to achieve at least a portion of step S430 as described in FIG. 4. For example, when a spectrum scanning phase is a portion of a perfusion scanning phase (and is not identical to the perfusion scanning phase), the processing device 120 or the data processing module 330 may generate a perfusion analysis result for a ROI of a target object based on single-energy scanning data and dual-energy scanning data by performing at least a portion of the process 700.

Step S710, a perfusion image of a ROI is generated based on first single-energy scanning data of a perfusion scanning phase other than a spectrum scanning phase and second single-energy scanning data of dual-energy scanning data. A detailed description of S710 can be referred to step S610.

Step S720, a spectrum image of the ROI is generated based on the dual-energy scanning data. A detailed description of S720 can be referred to step S620.

Step S730, a perfusion analysis result is obtained by performing a conjoint perfusion analysis on the spectrum image and the perfusion image.

The processing device 120 obtains a first analysis result by performing an analysis on the spectrum image, and obtains a second analysis result by performing a perfusion analysis on the perfusion image. Specifically, the processing device 120 processes the spectrum image using a spectrum analysis software, identifies spectrum features (e.g., an iodine concentration, a calcium content, or the like) of the ROI, and obtains the first analysis result of the ROI based on the spectrum features. For example, the first analysis result includes a tissue composition, a lesion nature, or the like. The processing device 120 obtains perfusion parameters (e.g., a blood flow, a blood volume, a time to peak, etc.) by performing a perfusion analysis on the perfusion image using the perfusion analysis software, and obtains the second analysis result of tissue hemodynamics of the ROI based on the perfusion parameters. Herein, a spatial resolution and a temporal resolution of the first analysis result and the second analysis result are matched for accurate conjoint analysis. Matching the spatial resolution and the temporal resolution of the first analysis result and the second analysis result means making the first analysis result and the second analysis result correspond to the same ROI at the same time point. For example, for a given anatomical level, the processing device 120 may obtain a blood flow (the second analysis result) at a given time point based on the perfusion image, and obtain an iodine concentration (the first analysis result) of an anatomical level at the time point based on the spectrum image, and perform a conjoint analysis on the blood flow and the iodine concentration.

The processing device 120 obtains the perfusion analysis result by performing a conjoint perfusion analysis on the first analysis result and the second analysis result. Specifically, the processing device 120 combines tissue composition information obtained from the analysis of the spectrum image with hemodynamic information obtained from the analysis of the perfusion image. Such a combination can be achieved, for example, by overlaying or fusing the two analysis results or by using statistical methods. Based on the fused data, the processing device 120 comprehensively evaluates the perfusion situation by taking into account the tissue composition information and the hemodynamic information, and ultimately obtains the perfusion analysis result (e.g., information on parameters such as an iodine concentration, a blood flow, a blood volume, etc.), or compares and analyzes the spatial distributions and variations of different parameters (e.g., an iodine concentration, a blood flow, a blood volume, etc.) to obtain the perfusion analysis result. The result obtained through the conjoint perfusion analysis combines information from the spectrum image and the perfusion image, and is capable of obtaining a variety of types of perfusion parameters, which improves the identification accuracy of the blood supply situation, lesions, etc., through a joint comparison of the perfusion parameters, thereby providing a comprehensive basis for clinical diagnosis and treatment.

In some embodiments, the conjoint perfusion analysis may be a fusion analysis of the low-energy data and the high-energy data (e.g., combining both the low-energy image data and the high-energy image data to perform the conjoint perfusion analysis). By fusing data from two energy levels, the accuracy and reliability of perfusion parameters can be improved. The conjoint perfusion analysis may also be a combination of the iodine concentration and the perfusion parameters. Combining an iodine concentration image with a perfusion parameter image can provide a comprehensive perfusion analysis result.

FIG. 8 is a flowchart illustrating an exemplary process of CT scanning according to some embodiments of the present disclosure. In some embodiments, at least a portion of a process 800 may be performed to achieve at least a portion of step S430 as described in FIG. 4. For example, when a spectrum scanning phase is identical to a perfusion scanning phase, the processing device 120 or the data processing module 330 may generate a perfusion analysis of a ROI of a target object based on single-energy scanning data and dual-energy scanning data by performing at least a portion of the process 800.

Step S810, fourth single-energy scanning data of a spectrum scanning phase is obtained based on dual-energy scanning data, and a perfusion image of a ROI is generated based on the fourth single-energy scanning data.

The dual-energy scanning data includes low-energy scanning data and high-energy scanning data, and the low-energy scanning data is the fourth single-energy scanning data. The processing device 120 extracts the low-energy scanning data from the dual-energy scanning data, and generates the perfusion image of the ROI by image reconstruction based on the low-energy scanning data. More content on generating the perfusion image based on the single-energy scanning data can be found in step S610, step S710, and the related descriptions thereof.

Step S820, a perfusion analysis result is obtained by performing a perfusion analysis on the perfusion image. A detailed description of step S820 can be referred to step S630.

In some embodiments of the present disclosure, the perfusion analysis result is obtained by performing the perfusion analysis on the perfusion image obtained based on the low-energy scanning data of the dual-energy scanning data when the spectrum scanning phase is identical to the perfusion scanning phase, which is capable of performing a spectrum CT scanning while performing a CT perfusion scanning, improving the scanning efficiency and ensuring the integrity of the perfusion analysis result.

FIG. 9 is a flowchart illustrating an exemplary process of CT scanning according to some embodiments of the present disclosure. In some embodiments, at least a portion of a process 900 may be performed to achieve at least a portion of step S430 as described in FIG. 4. For example, when a spectrum scanning phase is identical to a perfusion scanning phase, the processing device 120 or the data processing module 330 may generate a perfusion analysis result of a ROI based on single-energy scanning data and dual-energy scanning data by performing at least a portion of the process 900.

Step S910, a spectrum image of a ROI is generated based on dual-energy scanning data. A detailed description of step S910 can be referred to step S720.

Step S920, a perfusion analysis result is obtained by performing a perfusion analysis on the spectrum image.

The processing device 120 extracts a characteristic parameter of the spectrum image, and the characteristic parameter is used to characterize absorption properties of each substance in the ROI for rays of different energies. The processing device 120 establishes a time attenuation curve based on the characteristic parameter and obtains the perfusion analysis result by performing the perfusion analysis on the time attenuation curve. By establishing the time attenuation curve based on the spectrum image and performing the perfusion analysis on the time attenuation curve to obtain the perfusion analysis result, a variety of types of perfusion analysis results can be obtained, thereby reflecting the blood supply situation from different dimensions and enhancing the perfusion assessment effect.

The spectrum image includes a series of images of the same type (e.g., a sequence of images). For example, the spectrum image may include a series of virtual single-energy images, a series of iodine concentration distribution images, or a series of optimal signal-to-noise ratio images. Each image in the same series of images corresponds to a different time point, and series of virtual single-energy images may be used to capture dynamic changes in blood flow in the ROI.

In some embodiments, if the spectrum image is a virtual single-energy image, the processing device 120 extracts CT values (also referred to as density values) of all pixels in the ROI of the virtual single-energy image at each time point, and plots an average CT value (also referred to as an average density value) at each time point against the time point as the time attenuation curve. The processing device 120 obtains the perfusion analysis result by processing and analyzing the time attenuation curve using perfusion analysis software or algorithms. The perfusion analysis result may include perfusion parameters such as a blood flow, a blood volume, a mean transit time, and a time to peak.

In some embodiments, if the spectrum image is an iodine concentration distribution image, the processing device 120 may manually select one or more ROIs on an iodine concentration distribution image by a physician or a researcher, or may determine the ROIs using an automated segmentation algorithm. The processing device 120 extracts an average iodine concentration value at each time point in the ROI, and plots the average iodine concentration value at each time point against the time point as the time attenuation curve. The processing device 120 obtains the perfusion analysis result by processing and analyzing the time attenuation curve using perfusion analysis software or algorithms. The perfusion analysis result may include perfusion parameters such as a time to peak (e.g., a time to peak iodine concentration), a peak concentration (e.g., a highest iodine concentration value in the ROI), or the like.

In some embodiments, if the spectrum image is an optimal signal-to-noise ratio image, the processing device 120 extracts gray scale or density values of all pixels in the ROI of the optimal signal-to-noise ratio image at each time point, and plots an average value (or other statistic such as the median) of pixels in the ROI at each time point against the time point as the time attenuation curve. The processing device 120 obtains the perfusion analysis result by processing and analyzing the time attenuation curve using perfusion analysis software or algorithms. The perfusion analysis result may include perfusion parameters such as a blood flow, a blood volume, a mean transit time, a time to peak, or the like.

In some embodiments of the present disclosure, the perfusion analysis result is obtained by performing the perfusion analysis on the spectrum image generated based on the dual-energy scanning data when the spectrum scanning phase is identical to the perfusion scanning phase, which is capable of obtaining the perfusion analysis result without the perfusion image, improving the efficiency of perfusion analysis. At the same time, the dimensions of the perfusion analysis result are expanded to reflect the blood supply situation from different dimensions, improving the perfusion assessment effect.

FIG. 10 is a flowchart illustrating an exemplary process of CT scanning according to some embodiments of the present disclosure. In some embodiments, at least a portion of a process 1000 may be performed to achieve at least a portion of step S430 as described in FIG. 4. For example, when a spectrum scanning phase is identical to a perfusion scanning phase, the processing device 120 or the data processing module 330 may generate a perfusion analysis result of a ROI based on single-energy scanning data and dual-energy scanning data by performing at least a portion of the process 1000.

Step S1010, fourth single-energy scanning data of a spectrum scanning phase is obtained based on dual-energy scanning data, and a perfusion image of a ROI is generated based on the fourth single-energy scanning data. A detailed description of step S1010 can be referred to step S810.

Step S1020, a spectrum image of the ROI is generated based on the dual-energy scanning data. A detailed description of step S1020 can be referred to step S910.

Step S1030, a perfusion analysis result is obtained by performing a conjoint perfusion analysis on the spectrum image and the perfusion image. A detailed description of step S1030 can be referred to step S730.

In some embodiments of the present disclosure, the perfusion analysis result is obtained by performing the conjoint perfusion analysis on the spectrum image and the perfusion image generated based on the dual-energy scanning data when the spectrum scanning phase is identical to the perfusion scanning phase, which acquires a plurality of types of perfusion parameters by combining spectrum information with perfusion information, thereby improving the accuracy of identifying blood supply status and lesions through multiparameter joint comparison.

FIG. 11 is a flowchart illustrating an exemplary process of CT scanning according to some embodiments of the present disclosure. In some embodiments, at least a portion of a process 1100 may be performed to achieve at least a portion of step S430 as described in FIG. 4. For example, when a spectrum scanning phase is identical to a perfusion scanning phase, the processing device 120 or the data processing module 330 may generate a perfusion analysis result of a ROI based on single-energy scanning data and dual-energy scanning data by performing at least a portion of the process 1100.

Step S1110, spectrum data and perfusion data are obtained by performing a spectrum analysis and processing on dual-energy scanning data.

The processing device 120 performs the spectrum analysis and processing on the dual-energy scanning data using a spectrum analysis algorithm to extract information reflecting a substance composition as the spectrum data and information reflecting a blood supply as the perfusion data.

Step S1120, a spectrum perfusion image is obtained by fusing the spectrum data and the perfusion data.

The processing device 120 obtains a sequence of images (e.g., a sequence of spectrum images) reflecting tissue perfusion based on X-ray attenuation data at a single energy (e.g., low-energy data in the dual-energy scanning data) in the spectrum scanning phase. The processing device 120 obtains the spectrum perfusion image by fusing the spectrum data and the perfusion data and adding the spectrum data and the perfusion data to the spectrum image.

Step S1130, a perfusion analysis result is obtained by performing a perfusion analysis on the spectrum perfusion image.

The processing device 120 performs the perfusion analysis on the spectrum perfusion image using a similar approach to that used for a perfusion image. Specifically, the processing device 120 extracts a characteristic parameter in the spectrum perfusion image, and the characteristic parameter is used to characterize absorption properties of each substance in the ROI for rays of different energies. The processing device 120 constructs a time attenuation curve based on the characteristic parameter and performs the perfusion analysis on the time attenuation curve to obtain the perfusion analysis result. The perfusion analysis result obtained by the processing device 120 is a perfusion analysis result corresponding to the spectrum scanning phase. For example, assuming that the spectrum scanning phase is an arterial phase, the obtained perfusion analysis result is a perfusion analysis result of the arterial phase. More content on performing the perfusion analysis can be referred to steps S630, S820, S920, and the related descriptions thereof.

In some embodiments of the present disclosure, the perfusion analysis result is obtained by performing the perfusion analysis on the spectrum perfusion image obtained by fusing the spectrum data and the perfusion data based on the dual-energy scanning data when the spectrum scanning phase is identical to the perfusion scanning phase, and the information reflecting the substance composition and the information reflecting the blood supply can be directly fused into a same image. Therefore, it is not necessary to obtain the perfusion analysis result by reference and comparison between the two types of images (e.g., a spectrum image and a perfusion image), which improves analysis efficiency, and at the same time, the fusion of various information improves the accuracy of identifying blood supply conditions and lesions.

FIG. 12 is a flowchart illustrating an exemplary process of CT scanning according to some embodiments of the present disclosure. In some embodiments, a process 1200 may be executed by the CT system 100 and/or the CT system 300. For example, the process 1200 may be implemented as a set of instructions (e.g., an application) stored in a storage device (e.g., the storage device 130 illustrated in FIG. 1). In some embodiments, the processing device 120 of the CT system 100 and/or one or more modules of the CT system 300 may execute the set of instructions and may accordingly be directed to perform the process 1200.

Step S1210, in response to an operation of a user on an interactive interface of the CT system, a scanning instruction for a target object is generated. In some embodiments, step S1210 is performed by the instruction generation module 340.

After obtaining the operation (e.g., a mouse click, a keyboard input, a touch screen touch, etc.) of the user on the interactive interface of the CT system, the processing device 120 generates the scanning instruction for the target object based on the operation. The scanning instruction indicates a scanning mode set by the user, the scanning mode includes any of dual-energy scanning or a combination of single-energy scanning and dual-energy scanning. In some embodiments, the scanning mode includes a dual-energy scanning or a combination of a single-energy scanning and the dual-energy scanning. When the scanning mode is the dual-energy scanning, it indicates that the dual-energy scanning is performed in a plurality of phases throughout a scanning process. When the scanning mode is the single-energy scanning or a combination of the single-energy scanning and the dual-energy scanning, it indicates that the dual-energy scanning is performed in one or more phases throughout a scanning process, and the single-energy scanning is performed in the remaining phases of the one or more phases within the scanning process. In single-energy scanning, a radiation source (e.g., the first radiation source 210) that generates low energy X-rays works, and the corresponding single-energy scanning data (e.g., the low-energy data) acquired is used for CT perfusion imaging. In the dual-energy scanning, a radiation source (e.g., the first radiation source 210) that generates low energy X-rays and a radiation source (e.g., the second radiation source 220) that generates high energy X-rays work simultaneously, and the corresponding dual-energy scanning data acquired (including the low-energy data and the high-energy data) is used not only for CT perfusion imaging, but also for angiography and spectrum curve reconstruction.

For illustrative purposes only, FIG. 13 is a schematic diagram illustrating an exemplary interactive interface of a CT system according to some embodiments of the present disclosure. As shown in the interactive interface shown in FIG. 13, a scanning mode 1 is a single-energy scanning, and a scanning mode 2 is a dual-energy scanning. In each scanning mode, a user may set parameters such as scanning start time, scanning end time, a count of scanning periods (count of phases) of a scanning mode, an interval time between each phase, and a tube current-time product (mAs) by selecting or entering operations. The user may turn on a dual-energy scanning mode by selecting a button in the dual-energy scanning setting item. As shown in FIG. 13, the scanning settings with the serial numbers 1-3 indicate a consecutive scanning process, where the start time and end time of a scanning process with the serial number 1 are 5.0 S and 19.5 S, respectively, the start time and end time of a scanning process with the serial number 2 are 21.2 S and 26.0 S, respectively, and the start time and end time of a scanning process with the serial number 3 are 27.7 S and 34.2 S, respectively. In the scanning setting with the serial number 2, the button in the dual-scanning setting item is checked, and accordingly, the scanning mode is the scanning mode 2 (e.g., the dual-energy scanning). The scanning setting with the serial number 1 and the scanning setting with the serial number 3 are the scanning mode 1 (e.g., the single-energy scanning). By setting whether the dual-energy scanning is turned on, users can choose whether to perform a dual-energy scanning at each stage, thereby increasing adaptivity.

Step S1220, in response to determining that the target object is injected with a contrast agent, the CT system is controlled to execute the scanning instruction to obtain scanning data. In some embodiments, step S1220 is performed by the scanning module 320.

Corresponding to a single-energy scanning and a dual-energy scanning, respectively, the scanning data includes single-energy scanning data and dual-energy scanning data. The single-energy scanning data is the data acquired by the single-energy scanning, and the dual-energy scanning data is the data captured by the dual-energy scanning. An execution process of the scanning instruction (e.g., a scanning process) is divided into a plurality of phases according to a dose of contrast agent in an ROI of the target object (e.g., organs or tissues such as the heart, the brain, a bone, etc.). For example, the plurality of phases may include an inflow period, a peak period, and an outflow period. During at least one phase of the plurality of phases, the dual-energy scanning is performed to acquire the dual-energy scanning data. Or, during at least one first phase of the plurality of phases, the single-energy scanning is performed to obtain the single-energy scanning data, and during at least one second phase of the plurality of phases, the dual-energy scanning is performed to obtain the dual-energy scanning data.

Throughout the scanning process, the processing device 120 may perform the dual-energy scanning in all phases, with the two radiation sources of the CT system working simultaneously, which is then referred to as a dual-energy scanning across all phases. Or, throughout the scanning process, the processing device 120 may perform the dual-energy scanning in only a portion of a plurality of phases. During the phase of the dual-energy scanning, the processing device 120 controls the two radiation sources (e.g., the first radiation source 210 and the second radiation source 220) of the CT system to work simultaneously; and in the remaining phases of the phase of the dual-energy scanning within the scanning process, the single-energy scanning is performed, and during the phase of the single-energy scanning, the processing device 120 only controls the first radiation source (e.g., the first radiation source 210) of the CT system to work, which then referred to as a combination of the single-energy scanning and the dual-energy scanning.

Step S1230, an angiographic image of the at least one phase of the target object is obtained based on the dual-energy scanning data. In some embodiments, step S1230 is performed by the data processing module 330.

The processing device 120 obtains a target image by reconstruction based on the dual-energy scanning data, and the target image includes at least one of a high-energy angiogram, a low-energy angiogram, a virtual single-energy image, or a spectrum curve. For example, in the dual-energy scanning data, the processing device 120 may reconstruct a high-energy angiogram based on high-energy data corresponding to a radiation source that generates high energy X-rays. For another example, in the dual-energy scanning data, the processing device 120 may reconstruct a low-energy angiogram based on low-energy data corresponding to a radiation source that generates low energy X-rays. As another example, the processing device 120 may obtain a virtual single-energy image by combining the high-energy angiogram with the low-energy angiogram. As another example, the processing device 120 may obtain a spectrum curve for the high energy X-rays and a spectrum curve for the low energy X-rays by reconstruction based on the high-energy data and the low-energy data, respectively.

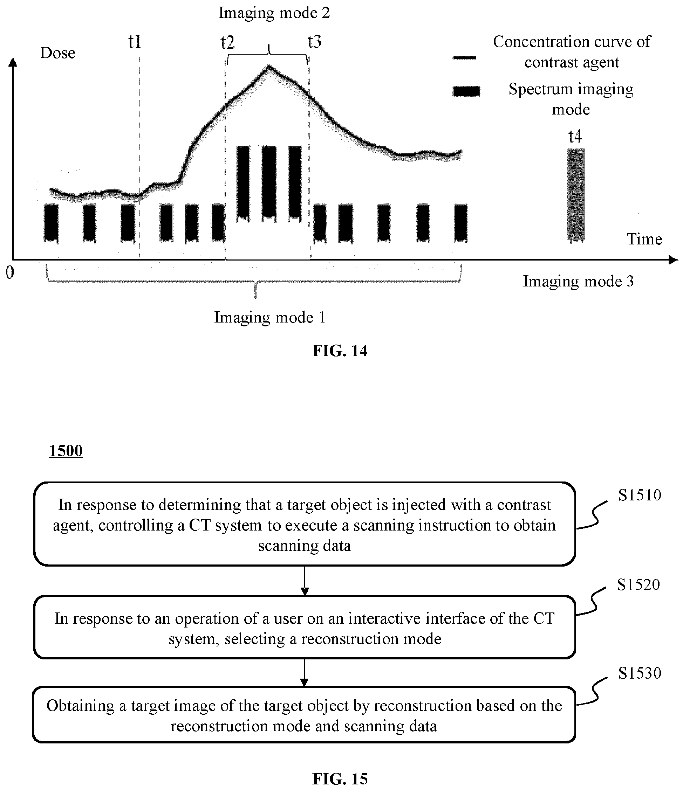

In some embodiments, the processing device 120 may also execute a delayed enhancement scanning instruction after the scanning instruction is executed. For illustrative purposes only, FIG. 14 is a schematic diagram illustrating an exemplary process of a CT system executing a scanning instruction according to some embodiments of the present disclosure. As shown in FIG. 14, a horizontal coordinate represents a time elapsed after a contrast agent is injected into a detection object (e.g., a target object); a vertical coordinate represents a dose of the contrast agent in a ROI of the target object; a curve represents a change of the dose of the contrast agent in the ROI with the time; bars show working voltages of radiation sources of the CT system, a lower bars corresponds to a single-energy scanning and represents two kinds of low-energy voltages, respectively, and a higher bars corresponds to a dual-energy scanning and represents a combined voltage of a high-energy voltage and a low-energy voltage. Depending on the dose of the contrast agent in the ROI of the detection object, the entire scanning process may be divided into four phases, including a non-contrast phase, an inflow phase, an arterial phase, and an outflow phase. The non-contrast phase corresponds to a period 0-t1; the inflow phase corresponds to a period t1-t2; the arterial phase corresponds to a period t2-t3; and the outflow phase corresponds to a period after t3. The processing device 120 performs a single-energy scanning in both the non-contrast phase and the inflow phase, at which time a radiation source of the CT system works at a first voltage; the processing device 120 performs a dual-energy scanning in the arterial phase, and the radiation sources of the CT system work at both a second voltage and a first voltage. Further, in the period t4, the processing device 120 further performs a delayed enhancement scanning, and the radiation source of the CT system also works at both the second voltage and the first voltage. Based on data corresponding to low energy X-rays of scanning data of the periods 0-t1, t1-t2, t2-t3, and the period after t3, the processing device 120 may perform reconstruction using an imaging mode 1 to obtain a full-phase perfusion image (a CTP image). Based on dual-energy scanning data of the period t2-t3, the processing device 120 may perform reconstruction using an imaging mode 2 to obtain an angiographic image (a CTA image). Based on dual-energy scanning data of a period t4, the processing device 120 may perform reconstruction using an imaging mode 3 to obtain a virtual single-energy image, or a spectrum curve for high energy X-rays, and a spectrum curve for low energy X-rays.

In conventional heart dynamic scanning, the scanning of the CTA image and the CTP image are often performed separately. First, an operator injects a first contrast agent into a target object; then, a CTP scanning is performed and reconstruction is performed; after the heart rate of the target object recovers and the contrast agent flows out, the operator continues to inject a second contrast agent into the target object, after which a CTA scanning is performed and reconstruction is performed. In some embodiments of the present disclosure, by switching a perfusion scanning in a combination of spectrum CT and CT perfusion scanning to a dual-energy scanning, it can simultaneously acquire a CTA image, a CTP image, and a spectrum image by a single injection of a contrast agent and a single scanning. At the same time, it can greatly reduce the amount of contrast agent used (e.g., by 50%), thereby reducing the dose of radiation to which the patient is subjected and the amount of contrast agent used; further, the scanning time can be dramatically reduced (e.g., by 75%), thereby increasing the efficiency of the examination and streamlining the workflow.

FIG. 15 is a flowchart illustrating an exemplary process of CT scanning according to some embodiments of the present disclosure. In some embodiments, a process 1500 may be executed by the CT system 100 and/or the CT system 200. For example, the process 1500 may be implemented as a set of instructions (e.g., an application) stored in a storage device (e.g., the storage device 130 illustrated in FIG. 1). In some embodiments, the processing device 120 of the CT system 100 and/or one or more modules of the CT system 300 may execute the set of instructions and may accordingly be directed to perform the process 1500. In some embodiments, the processing device 120 may implement the operations in steps S1220 to S1230 through at least a portion of the process 1500.

Step S1510, in response to determining that a target object is injected with a contrast agent, a CT system is controlled to execute a scanning instruction to obtain scanning data. A detailed description of step S1510 can be referred to step S1220.

Step S1520, in response to an operation of a user on an interactive interface of the CT system, a reconstruction mode is selected. In some embodiments, step S1520 and step S1530 are performed by the data processing module 330.

The reconstruction mode corresponds to a type of image obtained by reconstruction, and the reconstruction mode includes at least one of an angiography reconstruction mode, a perfusion imaging reconstruction mode, or a spectrum imaging reconstruction mode. For example, an angiographic image may be obtained by angiography reconstruction. As another example, a perfusion image may be obtained by perfusion imaging reconstruction. More content on responding to the operation of the user on the interactive interface of the CT system can be found in step S1210.

Step S1530, a target image of the target object is obtained by reconstruction based on the reconstruction mode and the scanning data.

The target image includes at least one of an angiographic image, a perfusion image, or a spectrum image.

Merely by way of example, when the reconstruction mode is the perfusion imaging reconstruction mode, the processing device 120 selects single-energy scanning data and low-energy data of dual-energy scanning data from the scanning data, and obtains a CTP image by reconstruction based on the low-energy data.

As another example, when the reconstruction mode is the spectrum reconstruction mode, the processing device 120 automatically determines a phase from a plurality of phases as a target phase, selects data corresponding to the target phase from the scanning data, and obtains a spectrum curve by reconstruction based on the data corresponding to the target phase. The target phase is also referred to as an optimal phase. For example, the user may select a phase to be collected or reconstructed using dual energy on a parameter interface in the interactive interface, and processing device 120 automatically select corresponding data for reconstruction based on the phase selected by the user.

As another example, when the reconstruction mode is the spectrum reconstruction mode, the processing device 120 selects data corresponding to a plurality of phases from the scanning data, and obtains the spectrum curve by reconstruction based on the data corresponding to a plurality of phases.

As another example, when the reconstruction mode is the angiography reconstruction mode, the processing device 120 obtains a high-energy CTA image by reconstruction based on high-energy data in the dual-energy scanning data, obtains a low-energy CTA image by reconstruction based on low-energy data in the dual-energy scanning data, and obtains a virtual single-energy image by combining the high-energy CTA image and the low-energy CTA image. More content on obtaining the target image can be found in S1230.

In some embodiments of the present disclosure, by performing an image reconstruction based on the operation of the user, the user may freely choose the angiography reconstruction mode, the perfusion imaging reconstruction mode, and the spectral imaging reconstruction mode, as desired. For example, for the angiography reconstruction mode, all phases may be selected. As another example, for the spectral imaging reconstruction mode, a single phase and a plurality of phases may be selected, and the single phase supports the selection of the optimal phase. At the same time, by switching a perfusion scanning in a combination of spectrum CT and CT perfusion scanning to a dual-energy scanning, a CTA image, a CTP image, and a spectrum image are obtained simultaneously with a single injection of a contrast agent and a single scanning process.

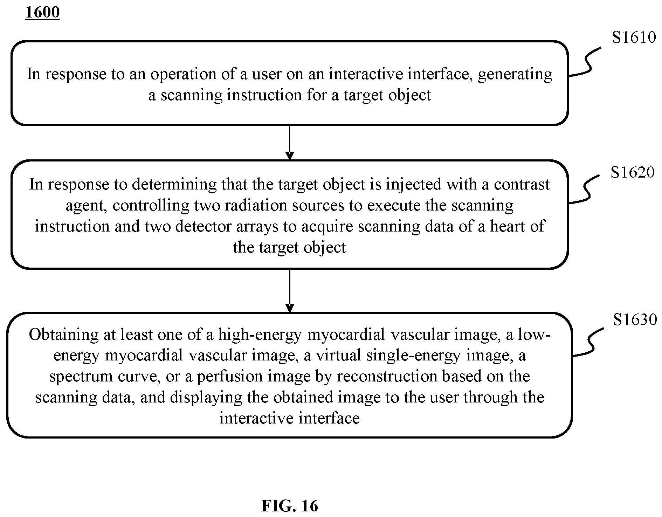

FIG. 16 is a flowchart illustrating an exemplary process of CT scanning according to some embodiments of the present disclosure. In some embodiments, a process 1600 may be executed by the CT system 100 and/or the CT system 200. For example, the process 1600 may be implemented as a set of instructions (e.g., an application) stored in a storage device (e.g., the storage device 130 illustrated in FIG. 1). In some embodiments, the processing device 120 of the CT system 100 and/or one or more modules of the CT system 300 may execute the set of instructions and may accordingly be directed to perform the process 1600.

Step S1610, in response to an operation of a user on an interactive interface, a scanning instruction for a target object is generated. A detailed description of step S1610 can be referred to step S1210.

Step S1620, in response to determining that the target object is injected with a contrast agent, two radiation sources (e.g., the first radiation source 210 and the second radiation source 220 shown in FIG. 2) are controlled to execute the scanning instruction and two detector arrays (e.g., the first detector array 230 and the second detector array 240 shown in FIG. 2) are controlled to acquire scanning data of a heart of the target object.

Depending on a dose of contrast agent in the heart of the target object, the entire scanning process is divided into four phases, including a non-contrast phase, an inflow phase, an arterial phase, and an outflow phase. In the non-contrast phase, the inflow phase, and the outflow phase, the processing device 120 all performs a single-energy scanning and controls a first radiation source (e.g., the first radiation source 210 shown in FIG. 2) of the CT system to work, and during the arterial phase, the processing device 120 performs a dual-energy scanning and controls the first radiation source and the second radiation source (e.g., the first radiation source 210 and the second radiation source 220 shown in FIG. 2) of the CT system to work simultaneously. Further, after the contrast agent flows out, the processing device 120 further performs a spectrum delayed enhancement scanning and controls the first radiation source and the second radiation source of the CT system to work simultaneously. More content on acquiring the scanning data can be found in steps S1220, S1510 to S1520, and the related descriptions thereof.

Although traditional myocardial CT perfusion imaging allows for a combined anatomical and functional assessment in a single session, in actual clinical practice, it is often difficult to capture a coronary vessel image during a perfusion scanning, resulting in inconsistent image quality of the coronary vessels. By setting spectrum CT imaging at certain phases during the arterial phase of the perfusion scanning, spectrum data of arterial vessels can be simultaneously acquired during the perfusion scanning. This approach enables high-quality vascular imaging using spectral technology in the combination of perfusion and angiographic imaging. Additionally, it allows for the comparison of changes in vascular iodine uptake and perfusion results within the same scan, providing multi-dimensional information to assist clinical diagnosis. The myocardial CT perfusion imaging based on low-energy data can improve vascular imaging quality. Multi-phase cardiac spectrum CT imaging allows the evaluation of myocardial ischemia using quantitative perfusion parameters and enables observation of changes in iodine uptake across different phases through iodine images, thereby assisting in tumor characterization (e.g., differentiating benign from malignant).

Step S1620, based on the scanning data, at least one of a high-energy myocardial vascular image, a low-energy myocardial vascular image, a virtual single-energy image, a spectrum curve, or a perfusion image is obtained, and the obtained image is displayed on the interactive interface.

Step S1630, at least one of the high-energy myocardial vascular image, the low-energy myocardial vascular image, the virtual single-energy image, the spectrum curve, or the perfusion image is obtained by reconstruction based on the scanning data, and the obtained image displayed to the user through the interactive interface of a terminal.

In some embodiments, the processing device 120 obtains at least one of structural information, functional information, or spectrum information of the heart of the target object based on the acquired images and displays the obtained information on the interactive interface. The functional information includes one or more of the following: a degree of coronary artery lumen stenosis, quantitative analysis results of coronary artery calcified plaques, and myocardial extracellular volume fraction (ECV).

In some embodiments, the processing device 120 may obtain perfusion images of all phases by reconstruction based on myocardial perfusion scanning data. The perfusion image can provide additional assessment of myocardial iodine content, thereby further establishing universal threshold values for normal, ischemic, and infarcted myocardium. The processing device 120 may obtain an angiographic image by reconstruction based on spectrum angiography scanning data. The angiographic image can achieve suppression of calcification and stent hardening artifacts, as well as enable the assessment of myocardial tissue perfusion defects. The processing device 120 may obtain a spectrum image by reconstruction based on data obtained from a delayed enhancement scanning. The evaluation of myocardial iodine delayed enhancement and the quantitative assessment of myocardial extracellular volume fraction (ECV) can be performed based on the spectrum image. Through the above reconstruction modes, spectral myocardial perfusion diagnosis is no longer affected by beam-hardening artifacts, thereby improving the detection rate of myocardial ischemia and enhancing the ability to differentiate between irreversible and reversible myocardial ischemia.

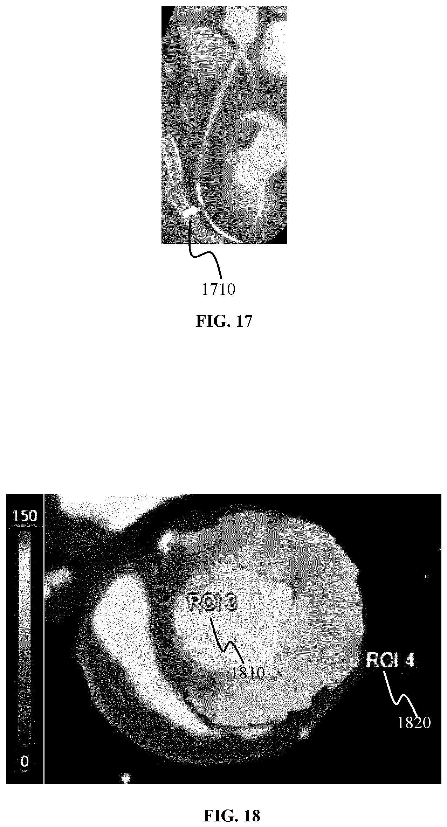

For example, FIG. 17 is a diagram illustrating an exemplary high-energy coronary computed tomography angiography (CTA) image according to some embodiments of the present disclosure. Arrow 1710 in FIG. 17 indicates distal occlusion of a left anterior descending (LAD) branch of a coronary artery.

As another example, FIG. 18 is a diagram illustrating an exemplary myocardial blood flow pseudo-color image according to some embodiments of the present disclosure. As shown in FIG. 18, different colors in the image represent quantitative values of absolute myocardial blood flow in various regions. In the color bar on the left side of the image, the top end at 150 is red, and the bottom end at 0 is purple. The color transitioning from red to purple, with the color gradually darkening, indicates a progressive decrease in the quantitative values. For example, in a region 1820 (a ROI 4), the absolute blood flow is 72.2, indicating the ROI region 4 is a normal myocardial region; whereas, in a region 1810 (a ROI region 3), the absolute blood flow is only 29.5, indicating that the myocardial region corresponding to the target object has a lower absolute blood flow than normal myocardium and the ROI 3 is an infarcted myocardial region.

The processing device 120 may also obtain a virtual single-energy image by reconstruction based on scanning data. The virtual single-energy image at low energy levels can improve the signal-to-noise ratio (SNR) and contrast-to-noise ratio (CNR) of the coronary artery CTA image, enhancing the image quality of the CTA image, which allows for achieving image quality equal to or better than that obtained with conventional contrast agent doses, while reducing the amount of intravenous iodine contrast agent injected. The virtual single-energy image at high energy levels can effectively reduce beam-hardening artifacts, halo artifacts, and image noise caused by subclavian vein contrast agent, sternal metallic wires, coronary artery bypass graft clips, and stents. This helps improve lumen visualization of severely calcified vessel walls, enhances the accuracy of coronary artery lumen stenosis assessment, and improves visualization within stents, thereby increasing the evaluation efficiency of the coronary artery stent lumen.

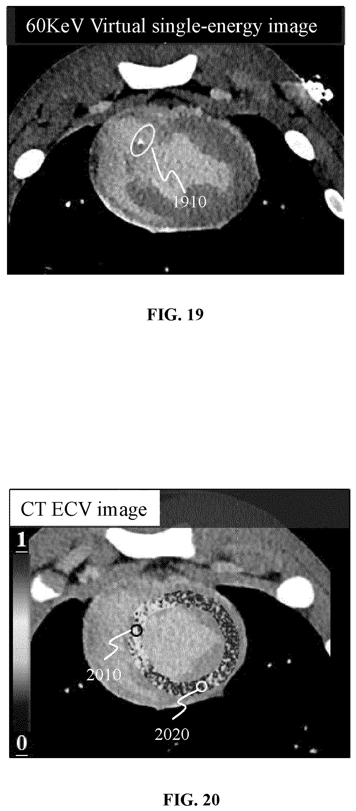

In some embodiments, the processing device 120 may further obtain a virtual single-energy image under delayed enhancement by reconstruction. Merely by way of example, FIG. 19 is a diagram illustrating an exemplary virtual single-energy image according to some embodiments of the present disclosure. The virtual single-energy image in FIG. 19 is obtained by performing a dual-energy scanning again on a target object during a specific period after a contrast agent inflow phase. This virtual single-energy image may correspond to a virtual single-energy level of 60 keV. In this image, delayed enhancement of infarcted myocardium can be visualized, as shown in a region 1910, which corroborates the scanning results in FIG. 17 and FIG. 18 (reconstructed images). The processing device 120 may further obtain a myocardial extracellular volume fraction (ECV) image by reconstruction. For example, FIG. 20 is a diagram illustrating an exemplary ECV image according to some embodiments of the present disclosure. As shown in FIG. 20, a region 2010, marked by a black circle in the image, represents the myocardial infarction area with an ECV value of 37.1%; and a region 2020, marked by a white circle, has an ECV value of 22.7%. It can be seen that the infarction region shown by the ECV results is consistent with the infarction regions displayed in FIGs. 17 to 19.

In one embodiment, the processing device 120 may further display dosage information on an interactive interface. For example, the interactive interface may include dosage display information. The dosage display information includes dosage information of at least one phase and/or a cumulative amount of the dosage that the target object has undergone. The dose display information enables an operator to grasp the radiation dose received by the target object in real time, thereby ensuring patient safety.

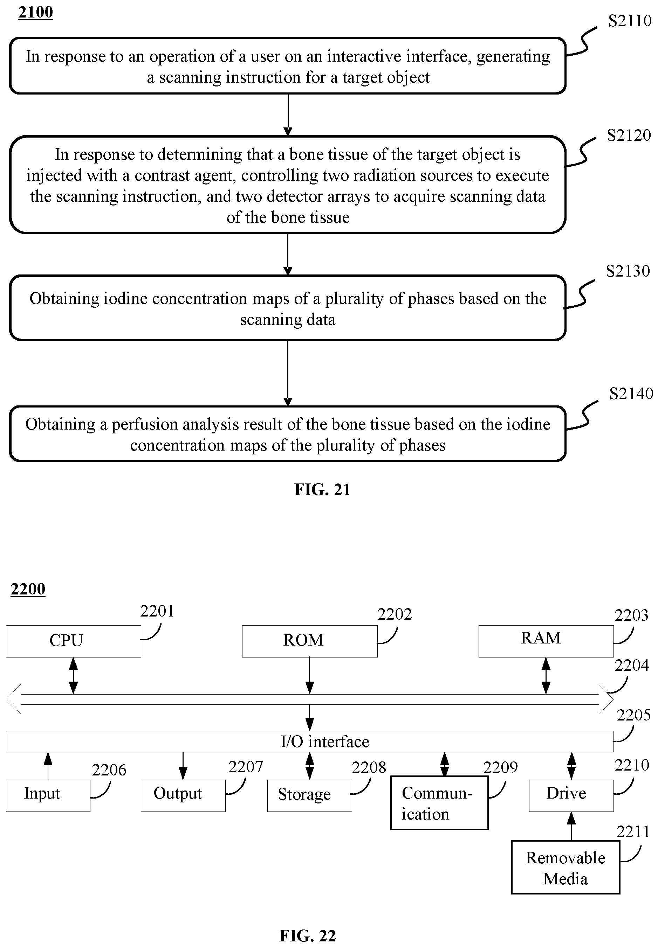

FIG. 21 is a flowchart illustrating an exemplary process of CT scanning on a bone tissue according to some embodiments of the present disclosure. In some embodiments, a process 2100 may be executed by the CT system 100 and/or the CT system 200. For example, the process 2100 may be implemented as a set of instructions (e.g., an application) stored in a storage device (e.g., the storage device 130 illustrated in FIG. 1). In some embodiments, the processing device 120 of the CT system 100 and/or one or more modules of the CT system 300 may execute the set of instructions and may accordingly be directed to perform the process 2100.

Step S2110, in response to an operation of a user on an interactive interface, a scanning instruction for a target object is generated. A detailed description of step S2110 can be referred to step S1210, and a scanning mode in step S2110 is a dual-energy scanning (e.g., a dual-energy scanning across all phases).

Step S2120, in response to determining that a bone tissue of the target object is injected with a contrast agent, two radiation sources are controlled to execute the scanning instruction, and two detector arrays are controlled to acquire scanning data of the bone tissue. A detailed description of step S2120 can be referred to step S1620, and the target object in step S2120 is the bone tissue.

Step S2130, iodine concentration images of a plurality of phases are obtained based on the scanning data. A detailed description of step S2130 can be found in steps S430, S620, S1530, and the related descriptions thereof.

Step S2140, a perfusion analysis result of the bone tissue is obtained based on the iodine concentration images of the plurality of phases. A detailed description of S2140 can be found in steps S430, S630, S730, S920, and the related descriptions thereof.

In the bone CT perfusion scanning, due to the relatively weak iodine enhancement in bone tissue and the small size of blood vessels, the enhanced CT values of conventional perfusion contrast agents are often difficult to distinguish from those of bone, making it challenging to identify the input of the CT values of the contrast agent during perfusion. In some embodiments of the present disclosure, by performing full-phase dual-energy scanning during bone CT perfusion imaging and obtaining iodine concentration images through calcium-iodine separation at each spectrum phase, the iodine images may be used for a perfusion analysis. This approach eliminates the influence of bone, overcomes the difficulty in distinguishing between bone CT values and contrast-enhanced CT values, and enhances the visualization of iodine input, thereby facilitating more accurate perfusion assessment.

The operations of the illustrated processes 400, 600, 700, 800, 900, 1000, 1100, 1200, 1500, 1600, and 2100 above are intended to be illustrative. In some embodiments, a process may be accomplished with one or more additional operations not described, and/or without one or more of the operations discussed. Additionally, the order in which the operations of a process described above is not intended to be limiting.

FIG. 22 is a schematic diagram illustrating an exemplary structure of a processing device according to some embodiments of the present disclosure. In some embodiments, the processing device 120 is implemented by a computer system 2200.

As shown in FIG. 22, the computer system 2200 includes a central processing unit (CPU) 2201, a read-only memory (ROM) 2202, a random-access memory (RAM) 2203, a bus 2004, and an I/O interface 2205, an input portion 2206, an output portion 2207, a storage portion 2208, a communication portion 2209, a driver 2210, and a removable media 2211.

The CPU 2201 performs a variety of appropriate actions and processes based on a program stored in the ROM 2202 or loaded into the RAM 2203 from the storage portion 2208. In the RAM 2203, various programs and data required to execute the instructions are also stored. The CPU 2201, the ROM 2202, and the RAM 2203 are connected to each other via the bus 2204. The I/O interface 2205 is also connected to the bus 2204.

Some of the components of the computer system 2200 are connected to the I/O interface 2205, i.e., the input portion 2206 including a keyboard, a mouse, or the like; the output portion 2207 including cathode ray tubes (CRTs), liquid crystal displays (LCDs), and speakers, or the like; the storage portion 2208 including hard disks, or the like; and the communication portion 2209 including network interface cards such as a LAN card, modem, or the like. The communication portion 2209 performs communication processing via a network such as the Internet. The driver 2210 is connected to the I/O interface 2205 as needed. The removable media 2211 is mounted to the driver 2210 as needed to allow computer programs read from it to be mounted into the storage section 2208 as needed. The removable media 2211 includes one or a combination of a disk, an optical disc, a magneto-optical disc, a semiconductor memory, or the like.

Operations described by the processes 400, 600, 700, 800, 900, 1000, 1100, 1200, 1500, 1600, and 2100 may be implemented as a computer software program and executed by the CPU 2201 to implement the operations of a scanning method according to some embodiments of the present disclosure.

Having thus described the basic concepts; it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the scope of the exemplary embodiments of this disclosure.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "unit," "module," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of a carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2103, Perl, COBOL 2102, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate $\pm 1\%$, $\pm 5\%$, $\pm 10\%$, or $\pm 20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A method for CT scanning, executed by a computing device including at least one processor and at least one storage device, the method comprising:

   obtaining a scanning instruction for a target object, wherein the scanning instruction is configured to instruct to perform a combination of spectrum CT and CT perfusion scanning on the target object, the scanning instruction includes scanning time information of the combination of spectrum CT and CT perfusion scanning, the scanning time information includes a spectrum scanning phase and a perfusion scanning phase, and the spectrum scanning phase is one or more scanning phases of the perfusion scanning phase; and
   in response to the scanning instruction, performing a CT perfusion scanning on the target object in the perfusion scanning phase other than the spectrum scanning phase, and performing a spectrum CT scanning on the target object in the spectrum scanning phase.

2. The method of claim 1, wherein the scanning instruction further includes energy information of the combination of spectrum CT and CT perfusion scanning, the energy information includes a single-energy voltage and a dual-energy voltage, and the performing the CT perfusion scanning on the target object in the perfusion scanning phase and the performing the spectrum CT scanning on the target object in the spectrum scanning phase includes:

   obtaining, using a perfusion scanning mode, single-energy scanning data of a region of interest (ROI) of the target object by emitting single-energy rays based on the single-energy voltage on the target object in the perfusion scanning phase other than the spectrum scanning phase, and
   obtaining dual-energy scanning data of the ROI of the target object by emitting dual-energy rays based on the dual-energy voltage on the target object in the spectrum scanning phase.

3. The method of claim 2, further comprising:

generating a perfusion analysis result of the ROI based on the single-energy scanning data and the dual-energy scanning data.

4. The method of claim 3, wherein the spectrum scanning phase is a portion of the perfusion scanning phase, and the generating a perfusion analysis result of the ROI based on the single-energy scanning data and the dual-energy scanning data includes:

generating a perfusion image of the ROI based on first single-energy scanning data in the perfusion scanning phase other than the spectrum scanning phase and second single-energy scanning data of the dual-energy scanning data;
generating a spectrum image of the ROI based on the dual-energy scanning data; and
obtaining the perfusion analysis result based on the spectrum image and the perfusion image, wherein the obtaining the perfusion analysis result includes:

obtaining the perfusion analysis result by performing a perfusion analysis based on the spectrum image and the perfusion image in the perfusion scanning phase other than the spectrum scanning phase; or
obtaining the perfusion analysis result by performing a conjoint perfusion analysis on the spectrum image and the perfusion image.

5. The method of claim 4, wherein the obtaining the perfusion analysis result by performing a perfusion analysis based on the spectrum image and the perfusion image in the perfusion scanning phase other than the spectrum scanning phase includes:

obtaining third single-energy scanning data by performing a scanning data conversion on the spectrum image; and
obtaining the perfusion analysis result by performing the perfusion analysis based on the first single-energy scanning data and the third single-energy scanning data.

6. The method of claim 3, wherein the spectrum scanning phase is identical to the perfusion scanning phase, and the generating a perfusion analysis result of the ROI based on the single-energy scanning data and the dual-energy scanning data includes:

obtaining fourth single-energy scanning data of the spectrum scanning phase based on the dual-energy scanning data and generating a perfusion image of the ROI based on the fourth single-energy scanning data; and/or
generating a spectrum image of the ROI based on the dual-energy scanning data.

7. The method of claim 6, further comprising:
obtaining the perfusion analysis result by performing a conjoint perfusion analysis on the spectrum image and the perfusion image.

8. The method of claim 6, further comprising: obtaining the perfusion analysis result by performing a perfusion analysis on the spectrum image or the perfusion image, wherein the obtaining the perfusion analysis result by performing a perfusion analysis on the spectrum image or the perfusion image includes:

extracting a characteristic parameter of the spectrum image, wherein the characteristic parameter characterizes absorption properties of each substance in the ROI for rays of different energies;
establishing a time attenuation curve based on the characteristic parameter; and
obtaining the perfusion analysis result by performing the perfusion analysis on the time attenuation curve.

9. The method of claim 3, wherein the spectrum scanning phase is identical to the perfusion scanning phase, and the generating a perfusion analysis result of the ROI based on the single-energy scanning data and the dual-energy scanning data includes:

obtaining spectrum data and perfusion data by performing a spectrum analysis and processing on the dual-energy scanning data;
obtaining a spectrum perfusion image by fusing the spectrum data and the perfusion data; and
generating the perfusion analysis result by performing a perfusion analysis on the spectrum perfusion image.

10. The method of claim 9, wherein the generating the perfusion analysis result by performing the perfusion analysis on the spectrum perfusion image includes:

extracting a characteristic parameter of the spectrum image, wherein the characteristic parameter characterizes absorption properties of each substance in the ROI for rays of different energies;
establishing a time attenuation curve based on the characteristic parameter; and
obtaining the perfusion analysis result by performing the perfusion analysis on the time attenuation curve.

11. A device for CT scanning, comprising at least one processor, wherein the at least one processor is configured to execute the method of any one of claims 1 to 10.

12. A method for CT scanning, executed by a computing device including at least one processor and at least one storage device, the scanning method comprising:

in response to an operation of a user on an interactive interface of a CT system, generating a scanning instruction for a target object, the scanning instruction indicating a scanning mode set by the user, and the scanning mode including dual-energy scanning or a combination of single-energy scanning and dual-energy scanning;
in response to determining that the target object is injected with a contrast agent, controlling the CT system to execute the scanning instruction to obtain scanning data, wherein an execution process of the scanning instruction is divided into a plurality of phases according to a dose of the contrast agent in a region of interest (ROI) of the target object, and during at least one phase of the plurality of phases, the dual-energy scanning is performed to acquire dual-energy scanning data; and
obtaining an angiographic image of the at least one phase of the target object based on the dual-energy scanning data.

13. A method for CT scanning, executed by a computing device including at least one processor and at least one storage device, the scanning method comprising:

in response to determining that a target object is injected with a contrast agent, controlling a CT system to execute a scanning instruction to obtain scanning data, wherein an execution process of the scanning instruction is divided into a plurality of phases according to a dose of the contrast agent in a region of interest (ROI) of the target object, during at least one first phase of the plurality of phases, single-energy scanning is performed to obtain single-energy scanning data, and during at least one second phase of the plurality of phases, dual-energy scanning is performed to obtain dual-energy scanning data, and the scanning data includes the single-energy scanning data and the dual-energy scanning data;
in response to an operation of a user on an interactive interface of the CT system, selecting a reconstruction mode, wherein the reconstruction mode includes at least one of an angiography reconstruction mode, a perfusion imaging reconstruction mode, or a spectral imaging reconstruction mode; and
obtaining a target image of the target object by reconstruction based on the reconstruction mode and the scanning data.

14. A system for CT scanning, the system comprising:

two radiation sources, wherein the two radiation sources include a first radiation source and a second radiation source, the first radiation source is configured to generate low energy X-rays based on a first voltage, and the second radiation source is configured to generate high energy X-rays based on a second voltage;
two detector arrays, corresponding to the two radiation sources, respectively, the two detector arrays being configured to receive X-rays transmitted through a target object and generate scanning data representing the X-rays transmitted through the target object;
a terminal, including an interactive interface configured to interact with a user; and
at least one processor and at least one storage device, the at least one storage device storing computer instructions, wherein when the computer instructions are executed by the at least one processor, the at least one processor executes following operations:

in response to an operation of the user on the interactive interface, generating a scanning instruction for the target object, the scanning instruction indicating a scanning mode set by the user, the scanning mode including dual-energy scanning or a combination of single-energy scanning and dual-energy scanning, wherein, during the single-energy scanning, the first radiation source works, and during the dual-energy scanning, the first radiation source and the second radiation source work simultaneously; and

in response to determining the target object is injected with a contrast agent, controlling the two radiation sources to execute the scanning instruction, and controlling the two detector arrays to acquire scanning data of a heart of the target object, wherein an execution process of the scanning instruction is divided into a plurality of phases according to a dose of the contrast agent in the heart, and during at least one phase of the plurality of phases, the dual-energy scanning is performed; and

obtaining at least one of: a high-energy myocardial vascular image, a low-energy myocardial vascular image, a virtual single-energy image, a spectrum curve, or a perfusion image based on the scanning data, and displaying the obtained image on the interactive interface.

15. A system for CT scanning, the system comprising:

two radiation sources, wherein the two radiation sources include a first radiation source and a second radiation source, the first radiation source is configured to generate low energy X-rays based on a first voltage, and the second radiation source is configured to generate high energy X-rays based on a second voltage;

two detector arrays, corresponding to the two radiation sources, respectively, configured to receive X-rays transmitted through a target object and generate scanning data representing the X-rays transmitted through the target object;

a terminal, including an interactive interface configured to interact with a user; and

at least one processor and at least one storage device, the at least one storage device storing computer instructions, wherein when the computer instructions are executed by the at least one processor, the at least one processor executes following operations:

in response to an operation of the user on the interactive interface, generating a scanning instruction for the target object, the scanning instruction indicating a scanning mode set by the user, the scanning mode including dual-energy scanning, wherein during the dual-energy scanning, the first radiation source and the second radiation source work simultaneously; and

in response to determining that a bone tissue of the target object is injected with a contrast agent, controlling the two radiation sources to execute the scanning instruction, and controlling the two detector arrays to acquire scanning data of the bone tissue, wherein an execution process of the scanning instruction is divided into a plurality of phases according to a dose of the contrast agent in the bone tissue;

obtaining iodine concentration images of the plurality of phases based on the scanning data; and

obtaining a perfusion analysis result of the bone tissue based on the iodine concentration images of the plurality of phases.

**100**

**FIG. 1**

**FIG. 2**

300

Obtaining module
310

Scanning module
320

Data processing module
330

Instruction generation module
340

**FIG. 3**

400

S410

Obtaining a scanning instruction for a target object

S420

In response to the scanning instruction, performing a CT perfusion scanning on the target object in the perfusion scanning phase other than the spectrum scanning phase, and performing a spectrum CT scanning on the target object in the spectrum scanning phase

S430

Generating a perfusion analysis result of a ROI based on single-energy scanning data and dual-energy scanning data

**FIG. 4**

510

Scanning setting interface
Perfusion scanning phase          Spectrum scanning phase
511                                512

Venous phase
Arterial phase ▼                   Arterial phase ▼

Venous phase √                     Venous phase

Arterial phase √                   Arterial phase √

●
●
●

OK      Cancel

**FIG. 5**

**600**

When a spectrum scanning phase is a portion of a perfusion scanning phase

Generating a perfusion image of a ROI based on first single-energy scanning data in the perfusion scanning phase other than the spectrum scanning phase and second single-energy scanning data of dual-energy scanning data — S610

Generating a spectrum image of the ROI based on the dual-energy scanning data — S620

Obtaining a perfusion analysis result by performing a perfusion analysis based on the spectrum image and the perfusion image in the perfusion scanning phase other than the spectrum scanning phase — S630

**FIG. 6**

**700**

When a spectrum scanning phase is a portion of a perfusion scanning phase

Generating a perfusion image of a ROI based on first single-energy scanning data of the perfusion scanning phase other than the spectrum scanning phase and second single-energy scanning data of dual-energy scanning data — S710

Generating a spectrum image of the ROI based on the dual-energy scanning data — S720

Obtaining a perfusion analysis result by performing a conjoint perfusion analysis on the spectrum image and the perfusion image — S730

**FIG. 7**

800

When a spectrum scanning
phase is identical to a
perfusion scanning phase

S810

Obtaining fourth single-energy scanning data of the spectrum scanning
phase based on dual-energy scanning data, and generating a perfusion
image of a ROI based on the fourth single-energy scanning data

S820

Obtaining a perfusion analysis result by performing a perfusion
analysis on the perfusion image

**FIG. 8**

900

When a spectrum scanning phase
is identical to a perfusion
scanning phase

S910

Generating a spectrum image of a ROI based on dual-energy scanning
data

S920

Obtaining a perfusion analysis result by performing a perfusion
analysis on the spectrum image

**FIG. 9**

**1000**

When a spectrum scanning
phase is identical to a
perfusion scanning phase

Obtaining fourth single-energy scanning data of the spectrum scanning
phase based on dual-energy scanning data, and generating a perfusion
image of the ROI based on fourth single-energy scanning data — S1010

Generating a spectrum image of the ROI based on the dual-energy
scanning data — S1020

Obtaining a perfusion analysis result by performing a conjoint
perfusion analysis on the spectrum image and the perfusion image — S1030

**FIG. 10**

**1100**

When a spectrum scanning
phase is identical to a
perfusion scanning phase

Obtaining spectrum data and perfusion data by performing a spectrum
analysis and processing on dual-energy scanning data — S1110

Obtaining a spectrum perfusion image by fusing the spectrum data and the
perfusion data — S1120

Obtaining a perfusion analysis result by performing a perfusion analysis on
the spectrum perfusion image — S1130

**FIG. 11**

1200

In response to an operation of a user on an interactive interface of the CT system, generating a scanning instruction for a target object

S1210

In response to determining that the target object is injected with a contrast agent, controlling a CT system to execute the scanning instruction to obtain scanning data

S1220

Obtaining an angiographic image of at least one phase of the target object based on dual-energy scanning data

S1230

**FIG. 12**

| No. | Starting time (S) | End time (S) | Interval | A count of scanning phases | Dual-energy scanning | mAs | Mode |
|---|---|---|---|---|---|---|---|
| 1 | 5.0 | 19.5 | 2.0S | 8 | ○ | 90 | Scanning mode 1 |
| 2 | 21.2 | 26.0 | Auto | 4 | ● | 80/164 | Scanning mode 2 |
| 3 | 27.7 | 34.2 | 2.0S | 4 | ○ | 90 | Scanning mode 1 |

**FIG. 13**

FIG. 14

FIG. 15

1600

In response to an operation of a user on an interactive interface, generating a scanning instruction for a target object
— S1610

In response to determining that the target object is injected with a contrast agent, controlling two radiation sources to execute the scanning instruction and two detector arrays to acquire scanning data of a heart of the target object
— S1620

Obtaining at least one of a high-energy myocardial vascular image, a low-energy myocardial vascular image, a virtual single-energy image, a spectrum curve, or a perfusion image by reconstruction based on the scanning data, and displaying the obtained image to the user through the interactive interface
— S1630

**FIG. 16**

1710

**FIG. 17**

**FIG. 18**

**FIG. 19**

**FIG. 20**

**2100**

S2110

In response to an operation of a user on an interactive interface, generating a scanning instruction for a target object

S2120

In response to determining that a bone tissue of the target object is injected with a contrast agent, controlling two radiation sources to execute the scanning instruction, and two detector arrays to acquire scanning data of the bone tissue

S2130

Obtaining iodine concentration maps of a plurality of phases based on the scanning data

S2140

Obtaining a perfusion analysis result of the bone tissue based on the iodine concentration maps of the plurality of phases

**FIG. 21**

**2200**

| 2201 | 2202 | 2203 |
|------|------|------|
| CPU | ROM | RAM |

2204

2205

I/O interface

| 2206 | 2207 | 2208 | 2209 | 2210 |
|------|------|------|------|------|
| Input | Output | Storage | Commun-ication | Drive |

2211

Removable Media

**FIG. 22**

| | Europäisches Patentamt European Patent Office Office européen des brevets | **EUROPEAN SEARCH REPORT** | Application Number EP 25 18 5160 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 106 923 856 A (FIRST AFFILIATED HOSPITAL ZHENGZHOU UNIV) 7 July 2017 (2017-07-07) | 1-7,9, 11,13 | INV. A61B6/50 A61B6/03 |
| Y | * abstract * | 12,14 | |
| A | * paragraph [0004] - paragraph [0007] * * paragraph [0017] * ----- | 8,10,15 | |
| Y | HAN SHUTING ET AL: "Pre-treatment spectral CT combined with CT perfusion can predict hemorrhagic transformation after thrombolysis in patients with acute ischemic stroke", EUROPEAN JOURNAL OF RADIOLOGY, ELSEVIER SCIENCE, NL, vol. 156, 24 September 2022 (2022-09-24), XP087222668, ISSN: 0720-048X, DOI: 10.1016/J.EJRAD.2022.110543 [retrieved on 2022-09-24] | 12 | |
| A | * abstract * * section "2. Materials and methods" * ----- | 1-11, 13-15 | TECHNICAL FIELDS SEARCHED (IPC) |
| Y | EP 3 602 487 B1 (KONINKLIJKE PHILIPS NV [NL]) 20 December 2023 (2023-12-20) | 14 | A61B |
| A | * abstract * * paragraph [0001] * * paragraph [0038] - paragraph [0039] * ----- | 1-13,15 | |

The present search report has been drawn up for all claims

| Place of search | Date of completion of the search | Examiner |
|---|---|---|
| Munich | 24 October 2025 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 25 18 5160

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2025

| Patent document cited in search report | | Publication date | Patent family member(s) | | Publication date |
|---|---|---|---|---|---|
| CN 106923856 | A | 07-07-2017 | NONE | | |
| EP 3602487 | B1 | 20-12-2023 | CN | 110506293 A | 26-11-2019 |
| | | | EP | 3602487 A1 | 05-02-2020 |
| | | | JP | 7191038 B2 | 16-12-2022 |
| | | | JP | 2020512133 A | 23-04-2020 |
| | | | US | 2020034968 A1 | 30-01-2020 |
| | | | WO | 2018178239 A1 | 04-10-2018 |

EPO FORM P0459

For more details about this annex : see Official Journal of the European Patent Office, No. 12/82

**EP 4 670 641 A1**

**Patent documents cited in the description**

- CN 202410834025